(19) Europäisches Patentamt
European Patent Office
Office européen des brevets

(11) **EP 3 573 748 B1**

(12) **FASCICULE DE BREVET EUROPEEN**


(45) Date de publication et mention de la délivrance du brevet:
**19.04.2023 Bulletin 2023/16**

(21) Numéro de dépôt: **18700697.8**

(22) Date de dépôt: **24.01.2018**

(51) Classification Internationale des Brevets (IPC):
***B01J 13/04*** *(2006.01)*

(52) Classification Coopérative des Brevets (CPC):
**B01J 13/04; A61P 39/00**

(86) Numéro de dépôt international:
**PCT/EP2018/051728**

(87) Numéro de publication internationale:
**WO 2018/138148 (02.08.2018 Gazette 2018/31)**


(54) **MATERIAUX HYBRIDES ORGANIQUES/INORGANIQUES COLORÉS ET PROCÉDÉ POUR LEUR PRÉPARATION**

FARBIGE ORGANISCH-ANORGANISCHE HYBRIDMATERIALIEN UND VERFAHREN ZUR HERSTELLUNG DAVON

COLOURED ORGANIC/INORGANIC HYBRID MATERIALS AND METHOD FOR PREPARING SAME


(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.01.2017 FR 1750613**

(43) Date de publication de la demande:
**04.12.2019 Bulletin 2019/49**



(73) Titulaires:
- **Centre National de la Recherche Scientifique**
  **75016 Paris (FR)**
- **Université Paul Sabatier Toulouse III**
  **31062 Toulouse Cedex 9 (FR)**

(72) Inventeurs:
- **MARTIN, François**
  **31570 Saint-Foy-d'Aigrefeuille (FR)**
- **LE ROUX, Christophe**
  **31290 Avignonet-Lauragais (FR)**
- **MICOUD, Pierre**
  **31390 Peyssies (FR)**
- **FERY-FORGUES, Suzanne**
  **82300 Monteils (FR)**
- **AYMONIER, Cyril**
  **33130 Bègles (FR)**
- **POIRIER, Mathilde**
  **26000 Valence (FR)**

(74) Mandataire: **Lavoix**
**2, place d'Estienne d'Orves**
**75441 Paris Cedex 09 (FR)**



(56) Documents cités:
**WO-A1-2014/049250 FR-A1- 2 792 322**
**FR-A1- 3 007 752**

- **YUFENG CHEN ET AL: "Structure and photoluminescence of amorphous silicate composites containing ZnO particles synthesized from layered sodium silicate", JOURNAL OF NON-CRYSTALLINE SOLIDS, vol. 358, no. 15, 2 juin 2012 (2012-06-02) , pages 1772-1777, XP028498083, ISSN: 0022-3093, DOI: 10.1016/J.JNONCRYSOL.2012.05.013 [extrait le 2012-05-18]**
- **FIGUEIREDO BRUNO R ET AL: "Photoluminescent porous and layered lanthanide silicates: A review", MICROPOROUS AND MESOPOROUS MATERIALS, vol. 234, 5 juillet 2016 (2016-07-05), pages 73-97, XP029706155, ISSN: 1387-1811, DOI: 10.1016/J.MICROMESO.2016.07.004**



Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).





Processed by Luminess, 75001 PARIS (FR)

## Description

La présente invention concerne des charges minérales nanométriques synthétiques de type phyllosilicates (silicates lamellaires) non gonflants telles que par exemple talc, mica, kaolinite, sur lesquelles sont adsorbés des composés organiques chargés, colorés. L'invention concerne également un procédé pour la préparation de ces charges et leurs utilisations.

## Etat de la technique antérieure

Les composés organiques colorés dans le visible sont connus pour de nombreuses applications, notamment pour le marquage, la coloration, la teinture et la détection des matériaux auxquels ils sont associés. Il peut s'agir de matériaux tels que des polymères, des particules minérales, des verres etc.... Certains matériaux colorés sont également utilisables dans le domaine cosmétique, et peuvent présenter à la fois des propriétés de coloration et de soin de la peau, comme par exemple les anthocyanines qui sont connues pour leur rôle d'agents antioxydants. Ainsi les anthocyanines sont utilisables en cosmétique pour prévenir ou retarder l'apparition des signes du vieillissement. Des applications de tels matériaux sont également connues dans les domaines de la catalyse ou de la papeterie.

Toutefois, certains composés organiques colorés peuvent être dégradés dans les conditions dans lesquelles ils sont mis en oeuvre et leurs propriétés s'en trouvent réduites. La coloration de matériaux par une molécule organique nécessite habituellement la formation d'une liaison covalente entre le composé à marquer et la molécule organique colorée. Une telle étape est souvent compliquée et coûteuse, elle est susceptible de modifier le comportement de la particule marquée.

Si certains talcs naturels (stéatites) présentent des couleurs variées telles que rose, gris ou vert, lorsqu'ils sont en forme de blocs tels qu'obtenus directement après extraction, leur broyage en fines particules (entre 2 et 200 μm) conduit irrémédiablement à l'obtention de poudres de couleur blanche à grisâtre, la couleur du talc naturel étant uniquement due à des agencements spécifiques des particules de talc les unes par rapport aux autres. Le broyage de tels talcs naturels conduit donc irrémédiablement à une perte de la coloration éventuellement rosée ou verdâtre naturelle initiale du talc.

Les particules minérales fonctionnalisées par au moins un groupement organique ou « particules hybrides organiques-inorganiques » présentent un intérêt croissant dans divers domaines de la chimie, de par leur capacité à combiner certains avantages des composés organiques et des composés inorganiques. La création d'interactions fortes entre des composés organiques et inorganiques permet une immobilisation durable d'espèces organiques sur des composés inorganiques, procurant aux espèces organiques l'ordre structural des composés inorganiques.

Il est connu de WO2014/049250 d'associer des particules minérales silicatées et des molécules colorantes pour donner des particules silicatées colorées. Les molécules colorantes mises en oeuvre dans ce document sont des cations métalliques. Les sels métalliques de métaux de transition adsorbés sur des phyllosilicates nanométriques confèrent à ceux-ci une coloration, mais de plus faible intensité qu'avec les colorants organiques

Les sels métalliques ont un comportement différent des molécules organiques, leurs propriétés respectives n'étant pas extrapolables d'une famille de composés à l'autre. Par exemple, on n'observe pratiquement aucune adsorption de sels métalliques sur des phyllosilicates de taille micronique, tandis que les molécules organiques colorées chargées peuvent s'adsorber partiellement sur des phyllosilicates de taille micronique. Toutefois, les inventeurs ont constaté de façon surprenante une adsorption beaucoup plus importante des molécules organiques colorées chargées sur des phyllosilicates nanométriques comparativement à des phyllosilicates micrométriques.

On connaît de WO2014/207397 un procédé de préparation d'une composition comprenant des particules minérales fonctionnalisées, dans lequel on met en contact une composition phyllosilicatée comprenant des particules minérales appartenant à la famille des silicates lamellaires, avec une solution comprenant un agent de fonctionnalisation hydrosoluble choisi dans le groupe formé des oxysilanes et des oxygermanes présentant au moins un groupement organique. Ce procédé requiert une étape de fonctionnalisation du groupement organique par un oxysilane et/ou un oxygermane. Les matériaux hybrides organiques-inorganiques ainsi obtenus ne permettent pas d'atteindre des taux de greffage satisfaisants.

Comme autre alternative à la préparation d'hybrides organiques-inorganiques, on connaît également des méthodes de synthèse directe de tels matériaux par voie sol-gel. Cependant, ces matériaux présentent des propriétés cristallines très faibles et des propriétés structurales très éloignées de celles des phyllosilicates naturels ou synthétiques non hybrides. En outre, ces synthèses par voie sol-gel ne peuvent en général pas être réalisées en milieu aqueux.

On connaît par le document WO2014/202920 un procédé de préparation d'une composition comprenant des particules minérales silico/germano-métalliques fonctionnalisées par au moins un groupement organique dans lequel on réalise un traitement hydrothermal d'un hydrogel précurseur des particules minérales silico/germano-métalliques. Le procédé se caractérise en ce que l'on utilise un hydrogel comprenant des particules silico/germano-métalliques fonctionnalisées par au moins un groupement organique.

On connaît de l'art antérieur V.M. Martinez, Langmuir 2004, 20, 5709-5717, des matériaux hybrides à base d'argile et de Rhodamine 6G. A la différence de l'invention, les argiles utilisées sont des matériaux gonflants. L'intercalation de Rhodamine 6G ou d'autres colorants dans une argile gonflante se produit par un mécanisme d'échange d'ions entre le ou les cation(s) interfoliaire(s) hydraté(s) et la molécule organique.

On connaît de WO2016/083404 des compositions comprenant un nanotalc de synthèse et des actifs cosmétiques, comme par exemple des filtres UV. On connaît de WO2016/083418 des compositions comprenant un nanotalc de synthèse et des actifs cosmétiques de type électrolytes ou polyélectrolytes. Toutefois, ces actifs ne sont pas des molécules colorées au sens de la présente invention. Par ailleurs, ces documents ne mentionnent pas la capacité des molécules colorees chargées à s'adsorber de façon irréversible sur un nanotalc de synthèse.

Le document Rahman A. et al., Water and Environnement Journal 29 (2015) 375-382 décrit l'utilisation de particules d'argile naturelle ou de synthèse pour retirer des colorants anioniques d'un milieu. L'application visée est la dépollution des effluents des industries textiles. Les matériaux utilisés sont micrométriques et présentent une faible cristallinité.

FR 2 792 322 décrit un procédé de fixation sur des particules minérales de molécules organiques de type quinonique ou indolique. Ce procédé permet de fixer de façon stable les molécules organiques sur les particules minérales, notamment pour les colorer ou leur conférer des propriétés de protection de l'épiderme contre les rayonnements ultraviolets.

L'invention vise à proposer un procédé permettant de préparer, de façon simple et rapide, une composition comprenant des particules minérales silicatées, les particules présentant des propriétés de coloration.

L'invention vise également à proposer un procédé de préparation d'une composition comprenant des particules minérales phyllosilicatées, les particules présentant des propriétés de coloration non seulement modulables selon la nuance et l'intensité de la couleur voulues mais également durables et stables dans le temps, à partir d'une composition comprenant des particules minérales phyllosilicatées déjà synthétisées.

Une telle composition capable de faire office à la fois de charge minérale fonctionnelle et de pigment, présente un intérêt majeur dans de nombreux domaines, tels que celui des cosmétiques, de la biologie, ou encore des charges minérales pour les peintures, des polymères, des matériaux optiques, du marquage biologique, ainsi que les encres. On peut également employer de telles compositions comme charge dans la composition de la pâte à papier.

L'invention vise à proposer un tel procédé dont la mise en oeuvre est simple et rapide, et est compatible avec les contraintes d'une exploitation industrielle.

L'invention a aussi pour objectif de proposer un procédé permettant de préparer une grande diversité chimique de compositions comprenant des particules minérales de structure et de propriétés similaires à celles des talcs, des micas ou des kaolinites naturels, et dont les propriétés de coloration peuvent être contrôlées et modifiées aisément.

La capacité des nanoparticules de phyllosilicates à fixer de façon irréversible des molécules organiques colorées chargées peut également être utilisée pour extraire d'un milieu des composés colorés.

## Résumé de l'invention

Un premier objet de l'invention concerne un procédé de préparation d'une composition hybride organique/inorganique comprenant des nanoparticules minérales fonctionnalisées par au moins une molécule choisie parmi les molécules organiques chargées colorées, ce procédé comprenant au moins la mise en contact, dans un milieu solvant monophasique, d'au moins une molécule organique chargée colorée et de nanoparticules de phyllosilicate non gonflant ayant une épaisseur de 1 nm à 100 nm, et une plus grande dimension de 10 nm à 10 µm.

Selon un mode de réalisation préféré, le procédé comprend au moins les étapes suivantes :

(i) Fourniture d'une solution (a) d'au moins une molécule organique chargée colorée dans au moins un solvant,
(ii) Fourniture d'une suspension (b) de nanoparticules de phyllosilicate non gonflant dans au moins un solvant,
(iii) Mise en contact de la solution (a) et de la suspension (b).

Selon un mode de réalisation avantageux, le procédé comprend en outre les étapes suivantes :

- Elimination de la phase solvant,
- Récupération des nanoparticules.

Selon un mode de réalisation préféré, le solvant de la solution (a) et le solvant de la suspension (b) sont miscibles.

L'invention concerne également une composition de nanoparticules hybrides organique/inorganique comprenant au moins un phyllosilicate non gonflant ayant une épaisseur de 1 nm à 100 nm, et une plus grande dimension de 10 nm à 10 µm ; et au moins une molécule choisie parmi les molécules organiques chargées colorées, ladite molécule organique étant adsorbée sur le phyllosilicate, cette composition étant susceptible d'être obtenue par le procédé selon

l'invention tel que décrit ci-dessus et de façon détaillée ci-dessous.

Les nanoparticules de phyllosilicate non gonflant présentent une épaisseur comprise entre 1 nm et 100 nm, et une plus grande dimension comprise entre 10 nm et 10 μm.

Selon un mode de réalisation préféré, les phyllosilicate non gonflant sont choisis parmi le talc, le mica, les kaolinites et leurs mélanges.

Selon un mode de réalisation préféré, les phyllosilicates non gonflants répondent à la formule chimique suivante :

■ $(SixGe(1-x))_4M_3O_{10}(OH)_2$, (I) dans laquelle :

- x est un nombre réel de l'intervalle [0 ;1],
- M désigne au moins un métal divalent ayant pour formule $Mg_{y1}Co_{y2}\,Zn_{y3}Cu_{y4}Mn_{y5}Fe_{y6}Ni_{y7}Cr_{y8}$; chaque indice yi représentant un nombre réel de l'intervalle [0 ; 1], et tel que $\sum_{i=1}^{8} yi = 1$.

Selon un autre mode de réalisation préféré, les phyllosilicates non gonflants répondent à la formule chimique suivante :

■ $(Aly'M'(1-y'))_2(Six'Ge(1-x'))_2O_5(OH)_4$, (II) dans laquelle :

- M' désigne au moins un métal trivalent choisi dans le groupe formé du gallium et des terres rares,
- y' est un nombre réel de l'intervalle [0 ; 1],
- x' est un nombre réel de l'intervalle [0 ; 1],

Selon encore un autre mode de réalisation préféré, les phyllosilicates non gonflants répondent à la formule chimique suivante :

■ $At(Six''Ge(1-x''))_4M''_kO_{10}(OH)_2$, (III) dans laquelle :

- A désigne au moins un cation monovalent d'un élément métallique ayant pour formule $Li_{w1}Na_{w2}K_{w3}Rb_{w4}Cs_{w5}$, chaque wi représentant un nombre réel de l'intervalle [0 ;1] tel que $\sum_{i=1}^{5} wi = 1$.
- x" est un nombre réel de l'intervalle [0 ;1],
- M" désigne au moins un métal divalent ayant pour formule $Mg_{j1}Co_{j2}\,Zn_{j3}Cu_{j4}Mn_{j5}Fe_{j6}Ni_{j7}Cr_{j8}$; chaque indice ji représentant un nombre réel de l'intervalle [0 ; 1], et tel que $\sum_{i=1}^{8} ji = 1$,
- k est un nombre réel de l'intervalle [2,50 ;2,85],
- t+2k est un nombre réel de l'intervalle [5,3 ;6,0].

Selon un mode de réalisation préféré, les phyllosilicates non gonflants sont formés d'un empilement de feuillets élémentaires de type phyllosilicate 2:1 et répondent à la formule chimique suivante :

■ $(SixGe(1-x))_4M_3O_{10}(OH)_2$, (I) dans laquelle :

- x est un nombre réel de l'intervalle [0 ;1],
- M désigne au moins un métal divalent ayant pour formule $Mg_{y1}Co_{y2}\,Zn_{y3}Cu_{y4}Mn_{y5}Fe_{y6}Ni_{y7}Cr_{y8}$; chaque indice yi représentant un nombre réel de l'intervalle [0 ; 1], et tel que $\sum_{i=1}^{8} yi = 1$.

Selon un autre mode de réalisation préféré, les phyllosilicates non gonflants sont formés d'un empilement de feuillets élémentaires de type phyllosilicate 1:1 et répondent à la formule chimique suivante :

■ $(Aly'M'(1-y'))_2(Six'Ge(1-x'))_2O_5(OH)_4$, (II) dans laquelle :

- M' désigne au moins un métal trivalent choisi dans le groupe formé du gallium et des terres rares,
- y' est un nombre réel de l'intervalle [0 ; 1],
- x' est un nombre réel de l'intervalle [0 ; 1],

Selon encore un autre mode de réalisation préféré, les phyllosilicatess non gonflants sont formées d'un empilement de feuillets élémentaires de type phyllosilicate 2:1 et répondent à la formule chimique suivante :

■ At(Six''Ge(1-x''))$_4$M''$_k$O$_{10}$(OH)$_2$, (III) dans laquelle :

- A désigne au moins un cation monovalent d'un élément métallique ayant pour formule $Li_{w1}Na_{w2}K_{w3}Rb_{w4}Cs_{w5}$, chaque wi représentant un nombre réel de l'intervalle [0 ;1] tel que $\sum_{i=1}^{5} wi = 1$ .
- x" est un nombre réel de l'intervalle [0 ; 1],
- M" désigne au moins un métal divalent ayant pour formule $Mg_{j1}Co_{j2}$ $Zn_{j3}Cu_{j4}Mn_{j5}Fe_{j6}Ni_{j7}Cr_{j8}$; chaque indice ji représentant un nombre réel de l'intervalle [0 ; 1], et tel que $\sum_{i=1}^{8} ji = 1$ ,
- k est un nombre réel de l'intervalle [2,50 ; 2,85],
- t+2k est un nombre réel de l'intervalle [5,3 ; 6,0].

Selon un mode de réalisation encore préféré, les phyllosilicates non gonflants sont formés d'un empilement de feuillets élémentaires :

■ de type phyllosilicate 2:1 et de formule chimique $Si_4M_3O_{10}(OH)_2$, plus particulièrement de formule chimique $Si_4Mg_3O_{10}(OH)_2$.

Selon un mode de réalisation encore préféré, les phyllosilicates non gonflants sont formés d'un empilement de feuillets élémentaires :

■ de type phyllosilicate 1:1 et de formule chimique $Al_2Si_2O_5(OH)_4$.

Selon un mode de réalisation encore préféré, les phyllosilicates non gonflants sont formés d'un empilement de feuillets élémentaires :

■ de type phyllosilicate 2:1 et de formule chimique K $Si_4$ Mg2,5 $O_{10}$ $(OH)_2$ (IIId) ou $K_{0,8}$ $Si_4$ $Mg_{2,6}$ $O_{10}$ $(OH)_2$ (IIIf)

Selon un mode de réalisation, la molécule organique colorée est choisie parmi celles présentant une absorption dans le domaine visible (longueur d'onde allant de 380 à 780 nm).

Selon un mode de réalisation, la solution (a) comprend en outre au moins une molécule organique photoluminescente chargée choisie parmi celles présentant une absorption dans le domaine ultraviolet (longueur d'onde allant de 200 à 380 nm) ou le visible (longueur d'onde allant de 380 à 780 nm), et ré-émettant l'énergie absorbée sous forme lumineuse.

Selon un mode de réalisation encore préféré, la molécule organique colorée est choisie parmi :

- les polyphénols, en particulier les anthocyanes, tels que la pélargonidine, la cyanidine, la péonidine, la delphinidine, la pétunidine, la malvidine, l'apigénidine, la lutéolidine, la tricétinidine, la 6-hydroxypélargonidine, la 6-hydroxy cyanidine, la 6-hydroxy delphinidine, l'europinidine, la rosinidine, la capensinidine, la pulchéllidine, l'hirsutidine, la 5-méthylcyanidine, la fisétinidine, ainsi que les mélanines telles que eumélanine et phéomélanine, et leurs dérivés, y compris les analogues synthétiques, les acides humiques,
- le bleu de méthylène ou chlorure de 3,7-bis(diméthylamino)phénothiazine-5-ylium, le cristal violet ou chlorure de tris (4-(dimethylamino)phenyl)methylium, le rouge congo ou acide benzidinediazo-bis-1-naphtylamine-4-sulfonique, les porphyrines, notamment la porphine, l'éosine Y ou acide bromofluorescéique, l'éosine B ou rouge impérial, l'orange thiazole ou p-tosylate de 1-méthyl-4-[(3-methyl-2(3H)-benzothiazolylidène)méthyl]quinolinium, l'Acid black 1 (CAS 1064-48-8), l'Acid black 2 (CAS 8005-03-6), le noir ériochrome T ou (4Z)-4-[(1-hydroxynaphthalén-2-yl-hydrazinylidène]-7-nitro-3-oxo Y-naphtalène-1-sulfonate de sodium, le bleu patenté V (CAS 20262-76-4 et 3536-49-0),
- un extrait aqueux d'une fraction végétale colorée, comme par exemple un extrait aqueux de raisin, de sureau, de grenade, d'açaï, de pétales de pavots, de mauves, de pivoines ou de betterave,
- un extrait aqueux d'une fraction animale colorée, comme par exemple un extrait de cochenille,
- un produit dérivé de ces extraits végétaux et animaux, comme par exemple du vin,
- les mélanges de ces composés.

Selon un mode de réalisation préféré, le ratio molécule organique colorée/phyllosilicate est de 0,001 % à 10 % en masse de carbone rapportée à la masse de phyllosilicate, de préférence de 0,01 % à 5 % en masse de carbone, rapportée à la masse de phyllosilicate.

Selon un mode de réalisation, la composition de nanoparticules hybrides est une composition solide, comme par exemple une poudre.

Selon ce mode de réalisation, de préférence la composition de nanoparticules hybrides consiste essentiellement en un ou plusieurs phyllosilicates non gonflants et une ou plusieurs molécules choisies parmi les molécules organiques chargées colorées.

Selon ce mode de réalisation, avantageusement la composition de nanoparticules hybrides consiste en un ou plusieurs phyllosilicates non gonflants et une ou plusieurs molécules choisies parmi les molécules organiques chargées colorées.

Selon un autre mode de réalisation, la composition de nanoparticules hybrides est une composition fluide, telle que par exemple une solution, une suspension, une dispersion ou un gel.

Selon ce mode de réalisation, avantageusement, la composition de nanoparticules hybrides comprend un ou plusieurs phyllosilicates non gonflants, au moins une molécule choisie parmi les molécules organiques chargées colorées et au moins un solvant choisi parmi l'eau, les solvants organiques, et les mélanges d'eau et de solvants miscibles avec l'eau.

Selon un mode de réalisation, la composition de nanoparticules hybrides consiste essentiellement en un ou plusieurs phyllosilicates non gonflants, une ou plusieurs molécules choisies parmi les molécules organiques chargées colorées et au moins un solvant choisi parmi l'eau, les solvants organiques, et les mélanges d'eau et de solvants miscibles avec l'eau.

Selon un mode de réalisation, la composition de nanoparticules hybrides consiste en un ou plusieurs phyllosilicates non gonflants, une ou plusieurs molécules choisies parmi les molécules organiques chargées colorées et un solvant choisi parmi l'eau, les solvants organiques, et les mélanges d'eau et de solvants miscibles avec l'eau.

L'invention a également pour objet l'utilisation de nanoparticules de phyllosilicate non gonflant, ayant une épaisseur de 1 nm à 100 nm, et une plus grande dimension de 10 nm à 10 $\mu$m, pour extraire d'un environnement une molécule organique chargée colorée, selon laquelle lesdites nanoparticules de phyllosilicate non gonflant forment, par adsorption de ladite molécule organique chargée colorée, une composition hybride organique/inorganique, étant entendu que lorsque ledit environnement est un tissu biologique, il s'agit d'un tissu biologique isolé ou cultivé.

La divulgation concerne aussi des nanoparticules de phyllosilicate non gonflant pour leur utilisation médicale pour extraire une molécule organique chargée colorée d'un individu ou d'un animal contaminé par une telle molécule.

La divulgation concerne aussi un procédé d'extraction d'une molécule organique chargée colorée à partir d'un environnement dans laquelle la molécule organique chargée colorée est présente, ce procédé comprenant au moins les étapes suivantes :

(1) Fourniture d'une suspension (b) de nanoparticules de phyllosilicate non gonflant,
(2) Mise en contact de la suspension (b) et de l'environnement,

étant entendu que lorsque ledit environnement est un tissu biologique, il s'agit d'un tissu biologique isolé ou cultivé.

Le procédé de l'invention présente l'avantage de permettre la formation du composite directement à partir d'une molécule organique colorée sans nécessiter de fonctionnalisation de cette molécule ou de la nanoparticule de phyllosilicate par des groupements métalliques ou par d'autres agents de liaison. La formation du composite repose en effet sur l'adsorption de la molécule organique chargée sur le phyllosilicate non gonflant. Ce procédé peut être mis en oeuvre en milieu aqueux ou organique. Il peut être mis en oeuvre à partir d'une composition de nanoparticules minérales avec différentes molécules colorées, sans qu'il soit nécessaire de former des précurseurs hybrides organique/inorganique préalablement à la production des nanoparticules. Cette façon de procéder confère à la méthode de l'invention plus de souplesse que certaines méthodes antérieures pour la préparation de matériaux de couleurs variées.

De façon surprenante, les inventeurs ont observé que des particules de phyllosilicate non gonflant, de dimension nanométrique, présentent une grande aptitude à adsorber des molécules organiques colorées dans la mesure où ces dernières sont porteuses de charges. La très forte adsorption des composés organiques colorés sur les nanocharges rend quasi impossible la diffusion des molécules colorées dans le milieu et conduit à des composés hybrides très stables. Ces propriétés sont d'autant plus surprenantes que, comparativement, les phyllosilicates de taille micronique montrent une capacité d'adsorption extrêmement faible.

L'association de molécules organiques chargées à des minéraux non gonflants lamellaires permet d'obtenir un matériau hybride organo-minéral présentant des propriétés de coloration choisies, modulables en intensité et en teinte.

Les nanoparticules de phyllosilicate non gonflant utilisées dans le procédé de l'invention présentent une capacité d'adsorption des molécules organiques colorantes chargées très supérieure à celle des phyllosilicates non gonflants naturels.

Par rapport aux phyllosilicates gonflants tels que certaines argiles, les nanoparticules de phyllosilicates non gonflants utilisées dans le procédé de l'invention présentent une coloration d'intensité comparable, avec une moindre quantité de colorant. Aussi, dans le cas de colorants coûteux, le rendu de couleur peut être identique à celui d'un composite à base d'argile gonflante, avec un coût de matière première bien inférieur.

Dans le cas où des marqueurs colorés toxiques sont utilisés en laboratoire, une manipulation maladroite peut conduire à un contact avec la peau ou les muqueuses du manipulateur, voire à une ingestion du produit. Une composition à base de nanoparticules de phyllosilicate peut, par sa capacité d'adsorption très forte de telles molécules, être utilisée pour fixer de façon irréversible de tels composés toxiques et faciliter leur élimination.

Dans la présente description, l'expression « compris entre X et Y » est entendue comme incluant les bornes, c'est-à-dire que le paramètre peut prendre les valeurs X, Y ou toute valeur entre X et Y.

L'expression « consiste essentiellement en » suivie d'une ou plusieurs caractéristiques, signifie que peuvent être inclus dans le procédé ou le matériau de l'invention, outre les composants ou étapes explicitement énumérés, des composants ou des étapes qui ne modifient pas significativement les propriétés et caractéristiques de l'invention.

Dans l'ensemble de la description, les termes « composite » et « hybride » sont employés indifféremment pour désigner un matériau mixte organique/inorganique.

Dans l'ensemble de la description, on désigne par « particule phyllominérale » toute particule minérale présentant une structure cristalline comprenant au moins une couche tétraédrique et au moins une couche octaédrique. Il peut s'agir par exemple de phyllosilicates.

Lorsque l'on indique que deux solvants sont miscibles, on entend qu'ils sont miscibles en toutes proportions lorsqu'ils sont à la température ambiante, c'est-à-dire à une température allant de 20°C à 25°C.

**Description détaillée**

Les inventeurs ont constaté avec surprise qu'un procédé selon l'invention permet de colorer une composition de phyllosilicate non gonflant de façon simple, rapide, mais néanmoins durable. Ainsi, une simple mise en contact de ladite composition de phyllosilicate dans une solution colorante comprenant des ions d'au moins un élément choisi parmi les molécules organiques colorées chargées, permet d'obtenir une composition comprenant des particules minérales phyllosilicatées colorées. Un tel procédé selon l'invention permet aussi d'obtenir une composition de particules minérales phyllosilicatées présentant des propriétés de coloration désirées à la façon d'un pigment, qui sont aisément ajustables en intensité et en nuance, qui permettent de couvrir toutes les couleurs du spectre visible, et restent ensuite stables dans le temps.

Le caractère surprenant de cette coloration réside notamment dans le fait qu'avec des particules différentes des particules minérales silicatées selon l'invention, de taille micrométrique, une très faible coloration des particules est obtenue. La durabilité et le caractère irréversible d'une telle étape de coloration restent à ce jour encore inexpliqués, de même que la facilité avec laquelle cette coloration est rendue possible.

Les nanoparticules de phyllosilicate

Les phyllosilicates sont des minéraux lamellaires largement répandus à la surface de la Terre. Ces minéraux sont constitués d'un empilement de feuillets suivant l'axe cristallographique c* et sont connus pour certains pour leur grande capacité d'adsorption.

Les phyllosilicates sont constitués par un empilement régulier de feuillets élémentaires de structure cristalline, dont le nombre varie de quelques unités à plusieurs milliers d'unités. Parmi les phyllosilicates (silicates lamellaires), le groupe comprenant notamment le talc, le mica et les smectites est caractérisé par le fait que chaque feuillet élémentaire est constitué par l'association de deux couches de tétraèdres situées de part et d'autre d'une couche d'octaèdres. Ce groupe correspond aux phyllosilicates 2:1. Au vu de leur structure, les phyllosilicates 2:1 sont également qualifiées de type T.O.T. (tétraèdre-octaèdre-tétraèdre). Le talc naturel, qui est un silicate de magnésium hydroxylé de formule $Si_4Mg_3O_{10}(OH)_2$, appartient à la famille des phyllosilicates.

La couche octaédrique des phyllosilicates 2:1 est formée de deux plans d'ions $O^{2-}$ et $OH^-$ (dans la proportion molaire $O^{2-}/OH^-$ de 2/1). De part et d'autre de cette couche médiane viennent s'agencer des réseaux bidimensionnels de tétraèdres dont un des sommets est occupé par un oxygène de la couche octaédrique, tandis que les trois autres le sont par des oxygènes sensiblement coplanaires.

Certains phyllosilicates, tels que les smectites, se caractérisent par la présence, entre les feuillets élémentaires, d'espaces interfoliaires qui renferment de l'eau et des cations et qui forment une phase gonflante du minéral. Les smectites sont donc qualifiées de type T.O.T. gonflant. Cette phase échangeable, gonflante, n'est pas présente dans les phyllosilicates non gonflants mis en oeuvre dans la présente invention.

Les micas se caractérisent notamment par la présence de cations interfoliaires dans des espaces, dits espaces interfoliaires, situés entre les feuillets élémentaires. À la différence des smectites, les micas sont dits non gonflants et

se caractérisent par l'absence de molécules d'eau dans les espaces interfoliaires, les molécules d'eau impliquant notamment une propriété de gonflement du minéral.

Comme cela est défini dans la publication scientifique intitulée « Nomenclature of Micas » de Rieder et al. (The Canadian Mineralogist, 1998, Vol. 36, pp 41-48), la formule simplifiée des micas est la suivante :

$$I\ M_{2\text{-}3\ \square 1\text{-}0}\ T_4\ O_{10}A_2$$

dans laquelle I désigne un cation interfoliaire (généralement K, Na ou Ca par exemple) ; M est généralement choisi parmi Li, Fe, Mg, Al ou encore Ti ; □ représente une vacance, T est généralement choisi parmi Al, Fe (trivalent) ou encore Si, et A est généralement choisi parmi F et OH notamment.

Les micas comprennent donc généralement de nombreux éléments chimiques dont du silicium, de l'aluminium ou encore du fer dans les sites tétraédriques (T dans la formule générale de Rieder et al.) et du lithium, du fer, du magnésium, de l'aluminium ou encore du titane dans les sites octaédriques (M dans la formule générale de Rieder et al.).

Les micas sont également caractérisés par une raie de diffraction aux rayons X caractéristique d'un plan (001) situé à une distance comprise entre 9,80Å et 10,30Å.

Les kaolinites appartiennent aussi à la famille des phyllosilicates. Parmi les phyllosilicates, le groupe comprenant notamment la kaolinite et la serpentine est caractérisé par le fait que chaque feuillet élémentaire est constitué par l'association d'une couche de tétraèdres et d'une couche d'octaèdres. La couche octaédrique des phyllosilicates 1:1 est formée d'un plan d'ions $O^{2-}$ et $OH^-$ (dans la proportion molaire $O_2^-/OH^-$ de 1/1). Chaque couche tétraédrique forme un réseau bidimensionnel de tétraèdres dont un des sommets est occupé par un oxygène de la couche octaédrique, tandis que les trois autres le sont par des oxygènes sensiblement coplanaires. Ce groupe correspond aux phyllosilicates 1:1. Au vu de leur structure, les phyllosilicates 1:1 sont également qualifiés de type T.O. (tétraèdre-octaèdre). Comme le talc et le mica, les kaolinites sont dites non gonflantes et se caractérisent par l'absence de molécules d'eau et de cations dans les espaces interfoliaires (espaces situés entre chaque feuillet élémentaire).

Selon l'invention on entend par « particules phyllosilicatées » des particules appartenant au groupe formé des silicates lamellaires, des germanates lamellaires, des germanosilicates lamellaires et de leurs mélanges.

Avantageusement l'invention concerne les silicates lamellaires.

Les phyllosilicates mis en oeuvre dans la présente invention appartiennent à la catégorie des phyllosilicates non gonflants.

Dans la présente invention, on désigne par « non gonflant(e) », tout phyllosilicate dont la raie de diffraction (001) n'est pas affectée par un traitement par mise en contact avec de l'éthylène glycol ou du glycol, c'est-à-dire dont la distance interatomique correspondant à la raie de diffraction (001) (aux rayons X) n'augmente pas après avoir été mis(e) en contact avec de l'éthylène glycol ou du glycol. Les phyllosilicates 2/ 1, à l'exception des smectites, sont non gonflants, il s'agit par exemple du talc ou d'autres phyllosilicates appartenant au groupe des micas tel que la muscovite. Les kaolinites également appartiennent à la catégorie des phyllosilicates non gonflants.

En particulier, la composition de nanoparticules de phyllosilicate utilisable dans le procédé de l'invention ne présente pas, en diffraction des rayons X, de raie de diffraction caractéristique d'un plan situé à une distance comprise entre 12,00 Å et 18,00 Å, caractéristique d'une phase gonflante.

De façon avantageuse, les phyllosilicates non gonflants utilisés dans la présente invention sont choisis parmi le talc synthétique, le mica synthétique, les kaolinites synthétiques et leurs mélanges.

De façon avantageuse, les phyllosilicates non gonflants utilisés dans la présente invention répondent à l'une des formules chimiques suivantes :

■ $(SixGe(1\text{-}x))_4M_3O_{10}(OH)_2$, (I) dans laquelle :

- x est un nombre réel de l'intervalle [0 ; 1],
- M désigne au moins un métal divalent ayant pour formule $Mg_{y1}Co_{y2}\ Zn_{y3}Cu_{y4}Mn_{y5}Fe_{y6}Ni_{y7}Cr_{y8}$ ; chaque indice yi représentant un nombre réel de l'intervalle [0 ; 1], et tel que $\sum_{i=1}^{8} yi = 1$ ,

  ou

■ $(Aly'M'(1\text{-}y'))_2(Six'Ge(1\text{-}x'))_2O_5(OH)_4$, (II) dans laquelle :

- M' désigne au moins un métal trivalent choisi dans le groupe formé du gallium et des terres rares,
- y' est un nombre réel de l'intervalle [0 ; 1],
- x' est un nombre réel de l'intervalle [0 ; 1],

  ou

■ $A_t(Si_{x''}Ge_{(1-x'')})_4M''_kO_{10}(OH)_2$, (III) dans laquelle :

- A désigne au moins un cation monovalent d'un élément métallique ayant pour formule $Li_{w1}Na_{w2}K_{w3}Rb_{w4}Cs_{w5}$, Li désignant le lithium, Na désignant le sodium, K désignant le potassium, Rb désignant le rubidium, Cs désignant le césium et chaque wi représentant un nombre réel de l'intervalle [0 ; 1] tel que $\sum_{i=1}^{5} wi = 1$.
- x" est un nombre réel de l'intervalle [0 ; 1],
- M" désigne au moins un métal divalent ayant pour formule $Mg_{j1}Co_{j2}\ Zn_{j3}Cu_{j4}Mn_{j5}Fe_{j6}Ni_{j7}Cr_{j8}$; chaque indice ji représentant un nombre réel de l'intervalle [0 ; 1], et tel que $\sum_{i=1}^{8} ji = 1$,
- k est un nombre réel de l'intervalle [2,50 ;2,85],
- t+2k est un nombre réel de l'intervalle [5,3 ;6,0].

De façon encore plus avantageuse, les phyllosilicates non gonflants utilisés dans la présente invention présentent au moins :

- une phase formée d'un empilement de feuillets élémentaires de type phyllosilicate 2:1 et de formule chimique $Si_4M_3O_{10}(OH)_2$, dans laquelle M a la même définition que ci-dessus, et plus particulièrement de formule chimique $Si_4Mg_3O_{10}(OH)_2$, ou
- une phase formée d'un empilement de feuillets élémentaires de type phyllosilicate 1:1 et de formule chimique $Al_2Si_2O_5(OH)_4$.

Selon une autre variante avantageuse, lorsque les phyllosilicates non gonflants utilisés dans la présente invention présentent au moins une phase formée d'un empilement de feuillets élémentaires de type phyllosilicate 2:1 et de formule chimique (III), l'une au moins des conditions suivantes est vérifiée :

- Avantageusement, ils sont exempts d'au moins un élément choisi parmi le fer, le zinc et le manganèse. En particulier, avantageusement, un phyllosilicate utilisé selon l'invention est exempt de fer.
- Dans une variante avantageuse de la formule (III), t est un nombre réel de l'intervalle [0,8 ; 1], k est un nombre réel de l'intervalle [2,5 ; 2,6].
- Dans une autre variante avantageuse de la formule (III), la formule (III) est telle que t + 2k = 6.
- Dans une variante avantageuse de la formule (III), x" = 1, le composé (III) ne comprend pas de Ge.
- Dans une variante avantageuse de la formule (III), le composé (III) peut comprendre, en sites octaédriques, un métal divalent de formule M". M" peut donc être choisi dans le groupe formé du magnésium, du cobalt, du zinc, du cuivre, du manganèse, du fer, du nickel et du chrome. Dans une variante particulièrement avantageuse d'un composé selon l'invention, M" est uniquement du magnésium (M"=Mg). Dans ce cas, le composé répond à la formule (IIIa) suivante :

$$A_t\ (Si_{x''}\ Ge_{(1-x'')})_4\ Mg_k\ O_{10}\ (OH)_2 \qquad \text{(IIIa)}$$

- Dans d'autres variantes avantageuses d'un composé (III), M" comprend du magnésium et au moins un autre métal tel que Mn ou Ni.
- En particulier, dans une variante de réalisation d'un composé (III), j3 est différent de 1. Plus particulièrement, dans une variante d'un composé (III), ledit composé est dénué de zinc et j3 est égal à zéro.
- En particulier, dans une variante de réalisation d'un composé (III), j6 est différent de 1. Plus particulièrement, dans une variante du composé (III), ledit composé est dénué de fer et j6 est égal à zéro. En effet, il peut être avantageux, dans certaines applications de disposer de composés dénués de fer ou dont la teneur en fer est limitée.
- Avantageusement et selon l'invention, dans la formule (III), A désigne au moins un élément chimique choisi dans le groupe formé du lithium (Li), du sodium (Na), du potassium (K), du rubidium (Rb) et du césium (Cs). Selon un mode de réalisation, A désigne l'un ou l'autre des éléments chimiques choisis dans le groupe formé du lithium (Li), du sodium (Na), du potassium (K), du rubidium (Rb) et du césium (Cs) (tel que w1 = 1, w2 = 1, w3 = 1, w4 = 1 ou w5 = 1 respectivement).

Dans la formule (III), A désigne un cation métallique disposé dans les espaces interfoliaires (entre les feuillets) dudit composé, chaque feuillet comprenant une succession d'une couche tétraédrique, d'une couche octaédrique puis d'une deuxième couche tétraédrique (soit une structure de type TOT, T désignant une couche tétraédrique et O désignant une couche octaédrique). Ainsi, avantageusement, un composé de formule (III) s'organise selon une structure solide formée de feuillets superposés les uns aux autres et séparés les uns des autres par au moins un espace, dit espace

interfoliaire, chaque cation A étant disposé dans lesdits espaces interfoliaires.

En particulier, avantageusement et selon l'invention, A est un cation interfoliaire non échangeable, c'est-à-dire qu'il est associé de façon stable et durable à la structure du mica synthétique. Cela signifie notamment qu'il reste associé audit composé lorsque le composé est mis en suspension dans de l'eau pure par exemple, ou dans de l'eau saturée en un cation différent de A, comme par exemple le calcium.

- Selon une variante particulièrement avantageuse d'un composé de formule (III), A désigne le potassium. Dans ce cas, le composé présente la formule (IIIb) suivante :

$$Kt\ (Six''\ Ge(1\text{-}x''))_4\ M_k\ O_{10}\ (OH)_2 \qquad (IIIb)$$

Conformément à la formule (III), le composé mica synthétique présente un coefficient stoechiométrique t relatif à la proportion stoechiométrique de A compris entre 0,30 et 1 (valeurs comprises), et en particulier entre 0,80 et 1 (valeurs comprises). Le coefficient stoechiométrique t est lié à la proportion stoechiométrique de métal M" dont le coefficient stoechiométrique est k.

- Dans une variante avantageuse d'un composé de formule (III), ledit composé est tel que t=1 et k=2,5 et présente la formule (IIIc) suivante :

$$A\ (Six''Ge(1\text{-}x''))_4\ M''_{2,5}\ O_{10}\ (OH)_2 \qquad (IIIc)$$

Lorsque, dans la formule (IIIc) précédente, A est le potassium, qu'aucun germanium ne se substitue au silicium et que M" est du magnésium, le composé selon l'invention présente la formule (IIId) suivante :

$$K\ Si_4\ Mg_{2,5}\ O_{10}\ (OH)_2 \qquad (IIId)$$

- Dans une autre variante avantageuse d'un composé de formule (III), ledit composé est tel que t=0,8 et k=2,6 et présente la formule (IIIe) suivante :

$$A_{0,8}\ (Six''Ge(1\text{-}x''))_4\ M''_{2,6}\ O_{10}\ (OH)_2 \qquad (IIIe)$$

Lorsque, dans la formule (IIIe) précédente, A est le potassium, qu'aucun germanium ne se substitue au silicium et que M" est du magnésium, le composé selon l'invention présente la formule (IIIf) suivante :

$$K_{0,8}\ Si_4\ Mg_{2,6}\ O_{10}\ (OH)_2 \qquad (IIIf)$$

Il est à noter qu'un composé de formule (III) est exempt d'aluminium et de fluor. En particulier, avantageusement, la composition de phyllosilicate est également exempte d'au moins un élément choisi parmi l'aluminium et le fluor. De telles compositions peuvent être particulièrement avantageuses dans certaines applications, par exemple des applications cosmétiques (en particulier en ce qui concerne l'aluminium).

Les phyllosilicates non gonflants utilisés dans le procédé de l'invention sont mis en oeuvre sous forme de nanoparticules. Avantageusement, on utilise des nanoparticules de dimension allant de 1 nm à 10 µm. De préférence, les nanoparticules de phyllosilicate utilisables dans l'invention présentent une épaisseur comprise entre 1 nm et 100 nm, et une plus grande dimension comprise entre 10 nm et 10 µm, l'épaisseur et la plus grande dimension étant évaluées par microscopie électronique.

Dans la présente invention, on désigne par « épaisseur » des particules minérales silicatées la plus petite dimension desdites particules, soit la dimension desdites particules selon la direction c* du réseau cristallin desdites particules minérales silicatées.

Dans la présente invention, on désigne par « plus grande dimension » des particules minérales silicatées, la dimension la plus grande desdites particules dans le plan (a, b) du réseau cristallin desdites particules minérales silicatées.

L'épaisseur et la plus grande dimension des particules minérales silicatées sont mesurées par observation par microscopie électronique à balayage (MEB) ou par microscopie électronique en transmission (MET) suivant des méthodes qui sont exposées dans la partie expérimentale.

Avantageusement et selon l'invention, lorsque l'on utilise les particules de formule (I), celles-ci présentent une épaisseur comprise entre 1 nm et 100 nm, en particulier entre 5 nm et 50 nm, et une plus grande dimension comprise entre 20 nm et 10 $\mu$m.

Avantageusement, lorsque l'on utilise des nanoparticules de formule (II), celles-ci présentent une taille moyenne comprise entre 20 nm et 600 nm, telle qu'observée par microscopie électronique.

Avantageusement et selon l'invention, lesdites particules minérales synthétiques de formule (II) présentent une épaisseur comprise entre 1 nm et 50 nm, en particulier entre 2 nm et 30 nm, par exemple de l'ordre de 10 nm.

Avantageusement et selon l'invention, la plus grande dimension des nanoparticules de formule (II) est comprise entre 10 nm et 600 nm, en particulier entre 20 nm et 500 nm et plus particulièrement entre 20 nm et 300 nm.

Avantageusement, lorsque l'on utilise des nanoparticules de formule (III), elles présentent une taille moyenne inférieure à 500 nm, notamment une taille moyenne comprise entre 10 nm et 400 nm (par exemple telle qu'observée par microscopie électronique).

Les particules de phyllosilicate de l'invention lorsqu'elles sont mises en présence d'un colorant organique chargé, permettent de former un matériau hybride phyllosilicate/colorant organique présentant une coloration intense.

Les inventeurs ont constaté que la taille des particules minérales silicatées est un facteur primordial pour permettre une telle coloration. Ainsi, les inventeurs ont observé que lorsque l'on applique un procédé selon l'invention à des particules de talc naturel présentant une épaisseur supérieure à 200 nm, l'épaisseur des talcs naturels les plus fins étant comprise entre 200 nm et 300 nm, seule une coloration de très faible intensité est obtenue.

Les nanoparticules de phyllosilicate non gonflant utilisables dans le procédé de l'invention peuvent être obtenues de la façon suivante :

- Procédé de préparation d'un phyllosilicate choisi parmi le talc :
  Dans le cas du talc synthétique, on peut procéder suivant les méthodes décrites dans les demandes WO2013/004979 et WO2015/159006.

La demande WO2013/004979 décrit un procédé de préparation d'une composition comprenant des particules minérales synthétiques, dans lequel on prépare un hydrogel précurseur desdites particules minérales synthétiques, par une réaction de co-précipitation entre :

- au moins un composé comprenant du silicium, et
- au moins un composé comprenant au moins un élément métallique, ladite réaction de co-précipitation ayant lieu en présence d'au moins un sel carboxylate de formule $R_2$-COOM" dans laquelle
- M" désigne un métal choisi dans le groupe formé de Na et K, et
- $R_2$ est choisi parmi H et les groupements alkyles comprenant moins de 5 atomes de carbone.

A titre de composé comprenant au moins un élément métallique, on peut utiliser tout composé métallique adapté pour réagir dans ladite réaction de co-précipitation dudit hydrogel précurseur desdites particules minérales synthétiques. Avantageusement, ledit composé comprenant au moins un élément métallique est un sel dicarboxylate de formule $M(R_1\text{-COO})_2$ dans laquelle :

- Ri est choisi parmi H et les groupements alkyles comprenant moins de 5 atomes de carbone et,
- M désigne au moins un métal divalent ayant pour formule $Mg_{y1}Co_{y2}\ Zn_{y3}Cu_{y4}Mn_{y5}Fe_{y6}Ni_{y7}Cr_{y8}$ ; chaque indice yi représentant un nombre réel de l'intervalle [0 ; 1], et tel que $\sum_{i=1}^{8} yi = 1$.

L'hydrogel est ensuite soumis à tout traitement adapté pour permettre d'obtenir lesdites particules minérales synthétiques de phyllosilicate, par exemple un traitement hydrothermal de type batch. Par exemple, on soumet ensuite l'hydrogel à un traitement hydrothermal à une température comprise entre 150°C et 400°C, pour obtenir lesdites particules de phyllosilicate.

Les groupements $R_1$ et $R_2$ peuvent être identiques ou différents, ils peuvent être choisis dans le groupe formé de $CH_3$-, $CH_3$-$CH_2$- et $CH_3$-$CH_2$-$CH_2$-.

Avantageusement, on utilise, à titre de composé comprenant du silicium, tout composé comprenant au moins un atome de silicium adapté pour réagir dans ladite réaction de co-précipitation dudit hydrogel précurseur desdites particules minérales synthétiques. En particulier, ledit composé comprenant du silicium est choisi dans le groupe constitué des silicates de sodium et des silices (dioxydes de silicium).

Avantageusement, on utilise du métasilicate de sodium à titre de composé comprenant du silicium. Avantageusement, ledit hydrogel précurseur desdites particules minérales synthétiques est un hydrogel silico/germano-métallique, de formule $(SixGe(1-x))_4M_3O_{11}$, $n'H_2O$ :

- x étant un nombre réel de l'intervalle [0 ;1],
- n' étant relatif à un nombre de molécule(s) d'eau associée(s) audit hydrogel silico/germano-métallique. Préférentiellement, ledit hydrogel silico/germano-métallique a pour formule $Si_4M_3O_{11}$, n'$H_2O$. Dans ce cas, ledit hydrogel silico/germano-métallique de formule $Si_4M_3O_{11}$, n'$H_2O$ est un hydrogel silicométallique.

Les particules minérales phyllosilicatées présentent au moins une phase non gonflante formée d'un empilement de feuillets élémentaires de type phyllosilicate 2:1 et de formule chimique $(Si_xGe_{(1-x)})_4M_3O_{10}(OH)_2$. En particulier, dans un mode de réalisation particulièrement avantageux d'un procédé selon l'invention, ladite phase non gonflante peut être formée d'un empilement de feuillets élémentaires de type phyllosilicate 2:1 et de formule chimique $Si_4M_3O_{10}(OH)_2$, et plus particulièrement de formule chimique $Si_4Mg_3O_{10}(OH)_2$ (M désignant alors le magnésium).

Au terme du traitement hydrothermal, on obtient une composition se présentant sous la forme d'une solution colloïdale renfermant des particules minérales phyllosilicatées présentant au moins une phase non gonflante.

A l'issue du traitement hydrothermal, on récupère une composition colloïdale comprenant des particules minérales synthétiques en suspension dans une solution aqueuse de sel(s) carboxylate(s). Ladite composition colloïdale peut ensuite être soumise à une étape de séchage, après une éventuelle étape de lavage à l'eau de façon à éliminer au moins en partie le(s)dit(s) sel(s) carboxylate(s). Une telle étape de lavage comprend au moins un cycle de lavage/centrifugation de ladite composition colloïdale.

Ladite composition comprenant des particules minérales synthétiques obtenue par un procédé selon l'invention peut être séchée par toute technique de séchage de poudre. Avantageusement, consécutivement audit traitement hydrothermal, on sèche lesdites particules minérales synthétiques obtenues par lyophilisation. Le séchage peut également être réalisé au moyen d'une étuve, par exemple à une température comprise entre 60°C et 130°C, pendant 1 heure à 48 heures, sous irradiation de micro-ondes, ou encore par atomisation.

En outre, il est possible de soumettre la composition comprenant des particules minérales synthétiques obtenue après traitement hydrothermal à un traitement thermique anhydre, dans l'air, à une température supérieure à 350°C et inférieure à la température de dégradation des particules minérales synthétiques. Avantageusement, on soumet une composition comprenant des particules minérales synthétiques obtenue après traitement hydrothermal à un traitement thermique anhydre, à une température comprise entre 350°C et 850°C, en particulier entre 400°C et 750°C et en particulier entre 450°C et 600°C, par exemple pendant une durée comprise entre 30 minutes et 24 heures. Avantageusement, après ledit traitement hydrothermal, on soumet la composition comprenant des particules minérales synthétiques à un traitement thermique anhydre. Un tel traitement thermique ou « recuit » permet une augmentation supplémentaire de la cristallinité des particules obtenues.

Selon cette variante, le procédé de l'invention fournit des nanoparticules de phyllosilicate caractérisées en ce qu'elles présentent, en diffraction des rayons X, des raies de diffraction caractéristiques suivantes :

- un plan (001) situé à une distance comprise entre 9,40 Å et 9,90 Å ;
- un plan (002) situé à une distance comprise entre 4,60 Â et 4,80 Â ;
- un plan (003) situé à une distance comprise entre 3,10 Â et 3,20 Â ;
- un plan (060) situé à une distance comprise entre 1,51 Â et 1,53 Â,

l'intensité de la raie de diffraction caractéristique d'un plan (002) étant supérieure à l'intensité du signal correspondant à un plan (020) situé à une distance comprise entre 4,40 Å et 4,60 Å, et, le rapport entre l'intensité de la raie de diffraction caractéristique d'un plan (001) et l'intensité de la raie de diffraction caractéristique d'un plan (003) étant compris entre 0,60 et 1,50.

La demande WO2015/159006 décrit un procédé de préparation de nanoparticules phyllosilicatées simple et rapide, compatible avec une exploitation à l'échelle industrielle, produisant des particules synthétiques phyllominérales de grande pureté, à caractère lamellaire, et présentant une granulométrie fine et de faible dispersion, ainsi qu'une structure cristalline très proches de celles des phyllominéraux naturels, notamment des phyllosilicates naturels, et en particulier du talc naturel. De telles nanoparticules sont utilisables dans le procédé de l'invention.

Le procédé de préparation de particules de phyllosilicates est réalisé par traitement solvothermal, d'un milieu réactionnel comprenant un milieu liquide et contenant en proportions stoechiométriques les éléments chimiques constitutifs desdites particules : on réalise ce traitement solvothermal en continu à une pression supérieure à 1 MPa et à une température comprise entre 100°C et 600°C, on fait circuler le milieu réactionnel en continu dans une zone, dite zone de traitement solvothermal, d'un réacteur continu avec un temps de séjour du milieu réactionnel dans la zone de traitement solvothermal adapté pour obtenir en continu, en sortie de la zone de traitement solvothermal, une suspension comprenant les particules de phyllosilicates.

De préférence, le traitement solvothermal est mis en oeuvre à une pression comprise entre 2 MPa et 50 MPa.

Le traitement solvothermal peut être appliqué à un gel précurseur comprenant les proportions stoechiométriques des éléments chimiques constitutifs des particules phyllosilicate, la transformation de ce gel précurseur produisant les

particules phyllominérales à l'issue du traitement solvothermal.

Avantageusement on utilise, à titre de gel précurseur, un hydrogel silico/germano-métallique précurseur, et on réalise ledit traitement solvothermal sous la forme d'un traitement hydrothermal en continu de cet hydrogel silico/germano-métallique précurseur.

Un hydrogel précurseur peut être obtenu, comme décrit ci-dessus suivant le procédé enseigné par WO2013/004979.

Selon cette variante, les particules phyllosilicatées utilisables dans le procédé de l'invention et obtenues par le procédé enseigné par WO2015/159006, présentent, en diffraction des rayons X, des raies de diffraction caractéristiques suivantes :

- un plan (001) situé à une distance comprise entre 9,40 Â et 12,50 Â ;
- un plan (003) situé à une distance comprise entre 3,10 Â et 3,30 Â ;
- un plan (060) situé à une distance comprise entre 1,51 Â et 1 ,53 Â.

Plus particulièrement, les particules phyllosilicatées obtenues par un procédé enseigné par WO2015/159006, présentent, en diffraction des rayons X, des raies de diffraction caractéristiques suivantes :

- un plan (001) situé à une distance comprise entre 9,40 Â et 12,50 Â ;
- un plan (002) situé à une distance comprise entre 4,60 Â et 5,00 Â ;
- un plan (003) situé à une distance comprise entre 3,10 Â et 3,30 Â ;
- un plan (060) situé à une distance comprise entre 1,51 Â et 1,53 Â.

- Procédé de préparation d'un phyllosilicate de type kaolinite :

Selon une seconde variante, les nanoparticules sont des kaolinites synthétiques et sont préparées par un procédé décrit dans la demande FR15/59125 dans lequel :

- on prépare un gel précurseur desdites particules minérales synthétiques de formule (II), par une réaction de co-précipitation entre :

    o au moins un sel d'un métal choisi dans le groupe formé de l'aluminium et de M', M' étant choisi parmi le fer, le galium et les terres rares,
    o au moins une source d'au moins un élément chimique choisi dans le groupe formé du silicium et du germanium, ladite source dudit élément chimique choisi dans le groupe formé du silicium et du germanium étant choisie dans le groupe formé du métasilicate de potassium, du métasilicate de sodium, du métagermanate de potassium et du métagermanate de sodium,
    o la proportion molaire (Aly'M'(1-y'))/(Six'Ge(1-x')) au cours de la préparation dudit gel précurseur étant égale à 1,

- on réalise un traitement solvothermal dudit gel précurseur à une température comprise entre 250°C et 600°C pendant une durée choisie de façon à permettre l'obtention de particules minérales synthétiques de formule (II).

En mettant en présence les réactifs ci-dessus et en respectant les proportions stoechiométriques du composé de formule (II) en ce qui concerne le rapport molaire entre l'aluminium et/ou M' et le silicium et/ou le germanium (Aly'M'(1-y'))/(Six'Ge(1-x')), on obtient un gel précurseur permettant d'obtenir, après traitement solvothermal, des particules minérales synthétiques de formule (II). Ce procédé permet d'obtenir un minéral synthétique non gonflant. Il n'est pas nécessaire de sécher le gel précurseur avant de réaliser le traitement solvothermal. Il n'est toutefois pas exclu de réaliser un tel séchage si l'on souhaite disposer ou conserver le gel précurseur sous la forme d'une poudre. En particulier, avantageusement, on réalise le traitement solvothermal dudit gel précurseur sans sécher préalablement le gel précurseur préparé.

Les particules minérales synthétiques obtenues par le procédé décrit ci-dessus ne gonflent pas en présence d'éthylène glycol ni de glycol. Les kaolinites synthétiques obtenues par ce procédé sont donc non gonflantes, comme les kaolinites naturelles, et présentent une charge électrique nulle.

Les kaolinites se caractérisent également par une grande stabilité thermique. Les particules minérales synthétiques obtenues par ce procédé présentent également une grande stabilité thermique, notamment jusqu'à 400°C. En particulier, avantageusement, une telle kaolinite synthétique est thermiquement stable jusqu'à 450°C (notamment dans l'air).

Avantageusement, les particules minérales synthétiques obtenues par le procédé décrit dans la demande FR15/59125 présentent, en diffraction des rayons X, au moins une raie de diffraction caractéristique d'un plan (001)

situé à une distance comprise entre 7,00 Å et 7,30 Å, notamment à une distance comprise entre 7,00 Å et 7,20 Å.

Les particules minérales synthétiques obtenues par le procédé décrit dans la demande FR15/59125 présentent, en moyen infrarouge quatre bandes de vibration entre 3610 $cm^{-1}$ et 3700 $cm^{-1}$, représentatives des vibrations d'élongation de groupements hydroxyle (-OH).

Tout réactif contenant de l'aluminium, du gallium ou un métal appartenant au groupe des terres rares, susceptible de permettre la préparation de particules minérales synthétiques selon la formule (II) (en particulier tout réactif pouvant être solubilisé dans un solvant, par exemple dans l'eau ou dans un alcool) peut être utilisé à titre de source d'aluminium ou de métal M' dans un procédé décrit dans la demande FR15/59125 de préparation des composés de formule (II). En particulier, avantageusement, ledit sel d'aluminium est choisi dans le groupe formé du sulfate d'aluminium, du nitrate d'aluminium, du chlorure d'aluminium et du phosphate d'aluminium.

Plus particulièrement, ledit sel d'aluminium est choisi dans le groupe formé du chlorure d'aluminium et du nitrate d'aluminium.

Dans une variante de réalisation avantageuse de ce procédé de préparation des composés de formule (II), M' désigne au moins un métal trivalent (c'est-à-dire présentant au moins un état d'oxydation de 3) choisi dans le groupe formé du fer, du gallium et des terres rares. En particulier, dans une variante de réalisation de ce procédé dans lequel M' comprend du fer, on réalise ledit traitement solvothermal en continu et pendant une durée inférieure à 12 heures, notamment inférieure à 6 heures.

Plus particulièrement, M' désigne au moins un métal ayant pour formule :

$$Ga_{z(1)}Sc_{z(2)}Y_{z(3)}La_{z(4)}Ce_{z(5)}Pr_{z(6)}Nd_{z(7)}Pm_{z(8)}Sm_{z(9)}Eu_{z(10)}Gd_{z(11)}Tb_{z(12)}Dy_{z(13)}Ho_{z(14)}Er_{z(15)}Tm_{z(16)}Yb_{z(17)}Lu_{z(18)}$$

dans laquelle chaque z(i) représente un nombre réel de l'intervalle [0 ; 1], tel que $\sum_{i=1}^{18} z(i) = 1$

Dans tout le texte, on désigne par terres rares les métaux choisis dans le groupe formé du scandium (Sc), de l'yttrium (Y), du lanthane (La), du cérium (Ce), du praséodyme (Pr), du néodyme (Nd), du prométhium (Pm), du samarium (Sm), de l'europium (Eu), du gadolinium (Gd), du terbium (Tb), du dysprosium (Dy), de l'holmium (Ho), de l'erbium (Er), du thulium (Tm), de l'ytterbium (Yb) et du lutécium (Lu).

Avantageusement, dans le procédé de production des composés de formule (II), une base, et en particulier une base forte, est ajoutée au cours de la réaction de coprécipitation dudit gel précurseur. Plus particulièrement, avantageusement et selon l'invention, au cours de la réaction de co-précipitation dudit gel précurseur, on ajoute une base choisie dans le groupe formé de NaOH (soude) et de KOH (potasse).

On réalise ledit traitement solvothermal pendant une durée permettant l'obtention de particules minérales synthétiques selon la formule (II). La durée du traitement solvothermal est choisie en fonction de la température et de la pression au cours du traitement solvothermal ainsi que des conditions dans lesquelles il est réalisé (batch, continu.) et éventuellement de la nature du solvant utilisé. En particulier, des particules minérales synthétiques selon la formule (II) peuvent être obtenues au bout de quelques minutes, voire au bout de quelques secondes de traitement solvothermal. La durée du traitement solvothermal est par exemple supérieure à 10 secondes et inférieure à 6 heures, et par exemple inférieure à 1 heure dans le cas d'une préparation en continu. En particulier, avantageusement, on réalise ledit traitement solvothermal pendant une durée inférieure à 48 heures, notamment inférieure à 24 heures. Plus particulièrement, on réalise ledit traitement solvothermal pendant une durée inférieure à 20 heures, notamment inférieure à 18 heures et par exemple inférieure à 12 heures.

Le traitement solvothermal peut être réalisé dans un réacteur fermé étanche (autoclave par exemple) ou encore de façon continue. Dans une variante particulièrement avantageuse, on réalise ledit traitement solvothermal en continu, en particulier en utilisant un réacteur continu. Tout réacteur continu connu peut être utilisé dans un procédé tel que décrit ci-dessus. Ainsi, avantageusement, ledit réacteur continu est un réacteur continu à volume constant. Dans une variante particulièrement avantageuse de ce procédé, on utilise un réacteur continu choisi dans le groupe formé des réacteurs piston (ou réacteurs à écoulement de type piston). Il peut par exemple s'agir de réacteurs tubulaires dans lesquels l'écoulement du milieu réactionnel s'effectue en régime laminaire, turbulent ou intermédiaire. En outre, il est possible d'utiliser tout réacteur continu à co-courant ou à contre-courant en ce qui concerne l'introduction et la mise en contact des différentes compositions et/ou milieux liquides mis en contact dans ce procédé.

On réalise le traitement solvothermal d'un milieu réactionnel comprenant ledit gel précurseur dans une zone de traitement solvothermal du réacteur à une température adaptée pour permettre l'obtention desdites particules synthétiques, en fonction notamment de la pression et de la durée du traitement solvothermal. Avantageusement et selon l'invention, on réalise ledit traitement solvothermal à une température comprise entre 280°C et 450°C. Plus particulièrement, avantageusement et selon l'invention, on réalise ledit traitement solvothermal à une température comprise entre 290°C et 420°C, notamment entre 290°C et 400°C, et en particulier entre 295°C et 375°C.

On réalise le traitement solvothermal d'un milieu réactionnel comprenant ledit gel précurseur dans une zone de traitement solvothermal du réacteur à une pression adaptée pour permettre l'obtention desdites particules synthétiques, en fonction notamment de la température et de la durée du traitement solvothermal. Avantageusement, on réalise ledit traitement solvothermal à une pression supérieure à 1 MPa. Plus particulièrement, on réalise ledit traitement solvothermal à une pression comprise entre 2 MPa et 50 MPa, notamment entre 8 MPa et 40 MPa, et en particulier entre 22 MPa et 30 MPa. Il s'agit en particulier de la pression de vapeur saturante à la température à laquelle est réalisé le traitement solvothermal, si le solvant est l'eau. Dans une variante particulièrement avantageuse de ce procédé, on réalise ledit traitement solvothermal en milieu aqueux. Il s'agit alors d'un traitement hydrothermal. L'eau peut être utilisée comme unique solvant ou diluant ou encore en mélange avec tout autre fluide.

Avantageusement et selon l'invention, il est possible d'utiliser comme formule chimique pour ledit gel précurseur, la formule chimique (IIbis) suivante :

$$2\ (Aly'M'(1\text{-}y'))\ 2\ (Six'Ge(1\text{-}x'))\ (5\text{-}\varepsilon)\ O\ (4+2\varepsilon)\ OH \qquad \text{(IIbis)},$$

dans laquelle $\varepsilon$ est un nombre réel de l'intervalle [0 ; 5[.
Une autre formule chimique est parfois également utilisée pour définir ledit gel précurseur, il s'agit de la formule suivante : $(Aly'M'(1\text{-}y'))_2\ (Six'Ge(1\text{-}x'))_2\ O_7$, ou encore en ce qui concerne un gel précurseur pour la préparation d'une kaolinite synthétique avec x'=1, y'=1 : $Al_2Si_2O_7$.

- Procédé de préparation d'un phyllosilicate de type mica :

Selon une troisième variante, les nanoparticules sont des micas et sont préparées par un procédé décrit dans la demande FR15/59129 comprenant les étapes suivantes :

1/ Préparation d'un gel précurseur d'un composé de formule (III)

Le gel précurseur d'un composé de formule (III) peut être préparé par une réaction de coprécipitation impliquant, à titre de réactif, au moins une source de silicium et/ou au moins une source de germanium, choisie(s) dans le groupe formé du métasilicate de potassium et du métagermanate de potassium, et au moins un sel métallique d'un métal divalent M", M" désignant au moins un métal divalent ayant pour formule $Mg_{j1}Co_{j2}\ Zn_{j3}Cu_{j4}Mn_{j5}Fe_{j6}Ni_{j7}Cr_{j8}$; chaque indice *ji* représentant un nombre réel de l'intervalle [0 ; 1], et tel que $\sum_{i-1}^{8} ji = 1$, (Mg désignant le magnésium, Co désignant le cobalt, Zn désignant le zinc, Cu désignant le cuivre, Mn désignant le manganèse, Fe désignant le fer, Ni désignant le nickel et Cr désignant le chrome) et chaque *ji* représentant un nombre réel de l'intervalle [0 ; 1], tel que $\sum_{i-1}^{8} ji = 1$.

Cette réaction de coprécipitation permet d'obtenir un gel précurseur présentant la stoechiométrie d'un mica synthétique répondant à la formule (III).

Le gel précurseur est préparé par une réaction de coprécipitation mise en oeuvre à partir de :

- une solution aqueuse dans laquelle est dissout au moins un sel métallique d'un métal divalent M", par exemple une solution aqueuse d'un sulfate métallique ($M''SO_4$),
- une solution d'acide sulfurique ($H_2SO_4$), et
- une solution aqueuse de métasilicate de potassium ou une solution aqueuse de métagermanate de potassium, ou un mélange de ces deux solutions dans les proportions molaires *x*"/(1-*x*").

La proportion molaire (Six"Ge(1-x"))/M" au cours de la préparation de ce gel précurseur est comprise dans l'intervalle [2/1,425 ; 1,6], et en particulier dans l'intervalle [2/1,3 ; 1,6].

La préparation de ce gel précurseur est réalisée en suivant le protocole suivant :
On mélange la solution comprenant au moins un sel métallique avec la solution d'acide sulfurique, on y ajoute ensuite la solution aqueuse de métasilicate de potassium et/ou de métagermanate de potassium ; le gel précurseur se forme instantanément.

La suspension obtenue comprenant le gel précurseur peut être soumise à une agitation à température ambiante (par exemple à 22,5°C) pendant 5 à 30 minutes puis être soumise à plusieurs cycles de lavage et de centrifugation ou être directement soumise à ces cycles de lavage et de centrifugation.

Le gel précurseur peut également être récupéré après centrifugation (par exemple entre 3000 et 15000 tours par minute, pendant 5 à 60 minutes) et élimination du surnageant (solution de sulfate de potassium) et lavage à l'eau déminéralisée (par exemple trois lavages et centrifugations successifs).

Le gel précurseur lavé et séparé de la solution comprenant du sulfate de potassium est ensuite soumis à un traitement solvothermal tel qu'obtenu à l'issue de la dernière centrifugation ou éventuellement après avoir été séché (par exemple dans une étuve ou encore par lyophilisation).

On ajoute ensuite audit gel précurseur au moins un hydroxyde de formule AOH de façon à ce que la proportion molaire A/M" soit au moins égale à t/k.

On obtient ainsi une suspension de gel précurseur et d'hydroxyde AOH.

2/ - Traitement solvothermal dudit gel précurseur

Le gel précurseur tel que précédemment obtenu (après l'ajout de l'hydroxyde AOH) est soumis à un traitement solvothermal à une température comprise notamment entre 300°C et 600°C.

Dans une première variante, le traitement solvothermal du gel précurseur est réalisé dans un réacteur fermé.

Pour ce faire on place le gel précurseur dans un réacteur/autoclave que l'on place à l'intérieur d'un four ou d'une étuve, à une température de réaction prédéterminée (établie entre 300°C et 600°C), pendant toute la durée du traitement solvothermal.

Au préalable, on ajuste éventuellement le rapport liquide/solide à une valeur comprise entre 2 et 80, notamment entre 5 et 50 (la quantité de liquide étant exprimée en $cm^3$, et la quantité de solide, en grammes, et désignant la quantité de gel sec uniquement).

En particulier, il est préférable de placer le réacteur ou l'autoclave dans les conditions de température et de pression du traitement solvothermal moins de 6 heures, notamment moins de 3 heures, et plus particulièrement moins d'une heure, après avoir ajouté l'hydroxyde de formule AOH au gel précurseur.

Au cours du traitement hydrothermal, le gel précurseur acquiert progressivement une consistance gélatineuse. La composition obtenue à l'issue du traitement solvothermal présente une cristallinité observable en diffraction des rayons X, cette cristallinité augmentant avec la durée du traitement solvothermal et se traduisant sur les diffractogrammes correspondants par l'apparition rapide de raies caractéristiques qui s'affinent et s'intensifient rapidement au cours du traitement.

À l'issue de ce traitement solvothermal, on obtient une composition comprenant des particules minérales de mica synthétique conforme à la formule (III) en suspension dans une solution, en particulier une solution aqueuse. Au terme de ce traitement solvothermal, la composition contenue dans le réacteur est récupérée par centrifugation (entre 3000 et 15000 tours par minute, pendant 5 à 60 minutes) puis élimination du surnageant.

La composition comprenant des particules minérales récupérée après la dernière centrifugation peut ensuite être séchée :

- à l'étuve à une température comprise entre 60°C et 130°C, pendant 1 à 24 heures, ou encore,
- par lyophilisation, par exemple dans un lyophilisateur de type CHRIST ALPHA® 1-2 LD Plus, pendant 48 heures à 72 heures,
- ou encore par atomisation.

Dans une deuxième variante, le traitement solvothermal du gel précurseur est réalisé en continu.

Dans un procédé dans lequel le traitement solvothermal est réalisé en continu, on utilise un réacteur 15 de préparation de particules minérales d'un composé selon l'invention en continu (tel qu'illustré à la figure 4) comprenant :

- une première portion 11 de conduit dans laquelle on introduit une première solution 20 aqueuse comprenant le gel précurseur,
- une deuxième portion 12 de conduit dans laquelle on introduit une deuxième solution 21 aqueuse comprenant au moins un hydroxyde de formule AOH (KOH par exemple),
- une troisième portion 13 de conduit disposée après la première portion 11 de conduit et la deuxième portion 12 de conduit et se prolongeant jusqu'à une entrée 9 d'une enceinte 16 de réaction, la première portion 11 de conduit et la deuxième portion 12 de conduit se rejoignant en un point 17 à partir duquel commence la troisième portion 13 de conduit,
- un conduit 14 réactionnel s'étendant à partir de l'entrée 9 dans l'enceinte 16 de réaction, et après la troisième portion 13 de conduit.

Une pompe péristaltique 18 permet d'alimenter en continu sous pression la première portion 11 de conduit avec la première solution 20 aqueuse contenue dans un réservoir 30 sous agitation. Une deuxième pompe péristaltique 19 permet d'alimenter en continu sous pression la deuxième portion 12 de conduit avec la deuxième solution 21 aqueuse contenue dans un réservoir 31 sous agitation.

Aux fins de contrôler la température au sein du conduit 14 réactionnel, l'enceinte 16 de réaction est un four

comprenant un manchon chauffant comprenant des résistances en matériau céramique. Le conduit 14 réactionnel est en forme générale de serpentin enroulé en de multiples spires à l'intérieur du manchon chauffant, jusqu'à sortir de cette dernière par une sortie 8 constituant la sortie de l'enceinte 16 de réaction.

Le mélange à l'intérieur de la troisième portion 13 de conduit est proche de la température ambiante. La troisième portion 13 de conduit est facultative, le point 17 et l'entrée 9 pouvant être confondus. Dans le mode de réalisation tel que représenté en figure 4 la troisième portion 13 de conduit présente par exemple une longueur comprise entre 10 cm et 20 cm.

Le temps de séjour total dans le dispositif pour préparer des particules synthétiques minérales par un procédé selon l'invention est inférieur à 30 minutes, et en particulier inférieur à 15 minutes voire de moins de 5 minutes ou de l'ordre d'une minute.

En outre, il est possible d'introduire d'autres solutions et notamment d'ajuster la quantité de solvant à différents niveaux du dispositif par exemple à l'aide d'entrées 4, 5 situées avant la zone de traitement solvothermal, l'entrée 4 étant située avant le point 17, l'entrée 6 étant située au niveau de la zone de traitement solvothermal, l'entrée 7 étant située après la sortie de la zone de traitement solvothermal et avant la sortie de la suspension obtenue.

Un régulateur 2 de pression est disposé en aval de l'enceinte 16 de réaction en liaison avec une cinquième portion 10 de conduit s'étendant de la sortie 8 du conduit 14 réactionnel et de l'enceinte 16 de réaction jusqu'à un récipient 25 dans lequel on récupère une suspension comprenant les particules minérales obtenues.

La fermeture d'une vanne 32 interposée sur la cinquième portion 10 de conduit permet de faire circuler la suspension obtenue à la sortie 8 du conduit 14 réactionnel dans un circuit 33 dérivé permettant de faire passer cette suspension à travers un fritté poreux 34 adapté pour retenir les particules et permettre leur récupération. Le fritté poreux 34 est plongé dans un bac à glace 35 permettant de refroidir la suspension sortant du réacteur. Dans ce cas, des vannes 36 et 37 disposées sur le circuit 33 dérivé sont ouvertes. Le fritté poreux 34 est choisi de façon à retenir les particules minérales synthétisées en les séparant du milieu liquide qui les transporte. Le fritté est par exemple réalisé en inox 316L, avec une taille de porosité de 50 $\mu$m. Lorsque le fritté 34 poreux est colmaté par des particules minérales, il suffit d'ouvrir la vanne 32 et de fermer les vannes 36 et 37, pour récupérer directement la suspension dans le récipient 25, cette suspension étant refroidie en passant par le bac à glace 35, puis lavée et centrifugée plusieurs fois pour récupérer les particules minérales qui peuvent être ensuite séchées, par exemple à l'étuve. Dans une autre variante (non représentée), il est bien sûr également possible de prévoir plusieurs frittés en parallèle, ce qui permet de diriger la suspension obtenue à la sortie du conduit 14 réactionnel vers un autre fritté dès que le précédent est colmaté par les particules minérales.

En variante, dans le cas où on prépare initialement une solution comprenant le gel précurseur et l'hydroxyde AOH, une même et unique portion de conduit remplace la première portion 11 de conduit et la deuxième portion 12 de conduit. Dans une autre variante, il est également possible que le réservoir 30 contienne une solution comprenant le gel précurseur et que le réservoir 31 contienne l'hydroxyde AOH.

Dans chaque cas, il est important de contrôler la dilution du gel précurseur introduit dans chaque portion de conduit et dans le conduit 14 réactionnel de façon à permettre une circulation en continu du milieu réactionnel dans le conduit 14 réactionnel, et dans l'ensemble des conduits d'amenée de ladite composition de gel précurseur jusqu'à l'entrée 9 de l'enceinte 16 de réaction. La concentration en gel précurseur dans ladite composition de gel précurseur introduite à l'entrée de l'enceinte 16 de réaction est avantageusement comprise entre $10^{-3}$ mol/L et plusieurs mol/L, par exemple de l'ordre de 0,01 mol/L. Cette concentration est beaucoup plus faible que les concentrations utilisées dans les procédés de préparation de particules synthétiques minérales telles que des phyllosilicates de l'état de la technique.

Le traitement solvothermal réalisé dans le conduit 14 réactionnel est un traitement solvothermal qui peut en particulier être réalisé dans des conditions supercritiques ou sous-critiques, et en particulier sous-critiques homogènes. Ainsi, on peut choisir la température et la pression auxquelles on réalise ce traitement solvothermal de façon à ce que la composition de gel précurseur introduite à l'entrée du réacteur, et en particulier le (ou les) solvant(s) qu'elle comprend, se trouve(nt) dans des conditions supercritiques ou dans des conditions sous-critiques homogènes, c'est-à-dire au-dessus de la courbe d'équilibre liquide-gaz du solvant, et de façon à ce que le solvant se présente à l'état liquide et non sous la forme d'un mélange liquide-gaz, ni de gaz seul.

À l'issue de ce traitement solvothermal, on obtient une suspension comprenant des particules minérales en solution, notamment en solution aqueuse. Au terme de ce traitement solvothermal, la suspension obtenue est récupérée par filtration, par exemple à l'aide d'un fritté en céramique, ou encore par centrifugation (entre 3000 et 15000 tours par minute, pendant 5 à 60 minutes) puis élimination du surnageant.

La composition comprenant des particules minérales récupérée peut éventuellement être lavée avec de l'eau, en particulier avec de l'eau distillée ou osmosée, en effectuant par exemple un ou deux cycles de lavage/centrifugation.

La composition comprenant des particules minérales récupérée après la dernière centrifugation peut ensuite être séchée :

- à l'étuve à une température comprise entre 60°C et 130°C, pendant 1 à 24 heures, ou encore,

- par lyophilisation, par exemple dans un lyophilisateur de type CHRIST ALPHA® 1-2 LD Plus, pendant 48 heures à 72 heures,
- par irradiation de micro-ondes,
- par atomisation,
- ou encore par toute autre technique de séchage de poudre.

La composition de particules minérales (b) issue d'un procédé solvothermal, avantageusement choisi parmi ceux décrits ci-dessus pour chacune des 3 variantes (talc, kaolinite, mica), peut être utilisée sous forme de suspension aqueuse directement issue du procédé solvothermal, avec éventuellement l'ajout d'un ou de plusieurs co-solvants. Les particules minérales peuvent aussi être dispersées dans un solvant organique, après séchage de la phase aqueuse, en vue de leur utilisation dans le procédé de l'invention. Parmi les solvants organiques utilisables, on peut mentionner des alcools ou des polyols, comme par exemple du méthanol, de l'éthanol, du glycérol, des cétones comme la propanone ou la 2-butanone.

Avantageusement, la composition de particules minérales (b) est sous forme d'une suspension aqueuse ou une suspension hydro-organique comprenant comme solvant un mélange d'eau et d'un ou plusieurs co-solvants tels que des alcools ou des polyols, comme par exemple du méthanol, de l'éthanol, du glycérol, des cétones comme la propanone ou la 2-butanone.

Encore plus avantageusement, la composition de particules minérales (b) utilisable dans le procédé de l'invention est une suspension aqueuse.

Molécule organique chargée colorée

Selon l'invention, on entend par molécule colorée ou molécule de colorant une molécule dont la coloration apparaît et est observable dans le domaine visible (longueur d'onde allant de 380 à 780 nm).

Selon un mode de réalisation, l'invention concerne des molécules colorées choisies parmi celles présentant une absorption dans le domaine visible (longueur d'onde allant de 380 à 780 nm).

Une molécule colorée ou molécule de colorant est toute molécule porteuse d'au moins un fragment chromophore présentant une absorption dans le domaine visible (longueur d'onde allant de 380 à 780 nm) et dont le coefficient d'extinction molaire $\varepsilon$ est supérieur ou égal à 1000 $m^2.mol^{-1}$, la mesure du coefficient d'extinction molaire précité étant effectuée à une température de 25°C dans l'eau, à la longueur d'onde correspondant à l'absorbance maximale de la molécule considérée et à une concentration à laquelle le colorant est totalement soluble dans le milieu .

On peut utiliser des mélanges de molécules chargées colorées.

Par molécule organique colorée on entend toute molécule qui comprend au moins un fragment chromophore. La molécule colorée peut être entièrement constituée d'un fragment chromophore.

Avantageusement, selon l'invention, le fragment chromophore de ladite molécule contient un nombre suffisant de doubles liaisons conjuguées induisant le pouvoir chromophore.

Le fragment chromophore de ladite molécule peut aussi contenir des hétéroatomes (B, N, O, S, P...) induisant ou modifiant le pouvoir chromophore.

Avantageusement, selon l'invention, le fragment chromophore de ladite molécule contient un nombre suffisant de doubles liaisons conjuguées pour conférer un caractère planaire ou sensiblement planaire au fragment chromophore de ladite molécule.

Les molécules colorées de l'invention présentent avantageusement un caractère planaire, c'est-à-dire qu'une partie au moins de ces molécules est plane ou sensiblement plane.

Par sensiblement plane on entend toute molécule (ou fragment de molécule) pour laquelle la déviation d'un ou de plusieurs atomes est inférieure à 15 picomètres par rapport au plan moyen, cette déviation pouvant être mesurée par diffraction de rayons X (mesure réalisée sur un monocristal) ou par suite d'une modélisation moléculaire dans le cas ou l'obtention d'un monocristal de ladite molécule n'a pu être possible, au moyen du logiciel i) Accelrys (Discovery Studio Modeling Environment, Release 4.0, San Diego: Accelrys Software Inc., 2013) et ii) Discovery Studio Visualizer 4.0 (DSV).

Par molécule comportant une partie plane, on entend une molécule dont au moins 4 atomes reliés entre eux sont placés dans le même plan.

Par molécule comportant une partie plane, on entend avantageusement une molécule dont au moins 4 atomes forment une structure comportant au moins deux liaisons multiples conjuguées.

Par liaisons multiples on entend des liaisons choisies parmi les liaisons doubles (par exemple C=C ou N=N) et les liaisons triples.

Selon un mode de réalisation, la molécule comportant une partie plane comprend au moins 5 atomes qui forment une structure comportant au moins deux liaisons multiples conjuguées et au moins un atome porteur d'un doublet d'électrons.

Selon un mode de réalisation, la molécule comportant une partie plane comprend au moins 6 atomes qui forment un cycle aromatique.

Les molécules de type polycyclique aromatique forment un système Π conjugué et sont planes. Les molécules hétérocycliques aromatiques conjuguées présentent généralement une structure sensiblement plane.

Par molécule chargée on entend :

i) toute molécule (ou fragment de molécule) porteuse d'au moins une fonction ionisable ou ionisée

- sous la forme d'un sel avec un métal choisi de préférence parmi les métaux alcalins, alcalino-terreux, les métaux de transition et les terres rares ou leurs mélanges, ou
- sous la forme d'un sel organique tels que les sels d'acides carboxyliques ou d'acides sulfoniques ;

Ainsi que
ii) toute molécule (ou fragment de molécule) porteuse d'au moins un hétéroatome (N, P, S, O) ionisé, comme par exemple la berbérine ou le bleu de méthylène,
iii) toute molécule porteuse d'une ou plusieurs fonctions ionisables ou ionisées et comprenant un ou plusieurs hétéroatomes ionisé(s).

Par fonction ionisable on entend une fonction acide carboxylique, acide phosphonique, une fonction $C-SO_3H$, ainsi que les phénols. Le fragment chargé de la molécule peut éventuellement être distinct du fragment chromophore de ladite molécule.

Parmi les molécules colorées visibles utilisables dans le procédé de l'invention, on peut citer :
Les anthocyanes : les anthocyanes font partie de la famille des polyphénols et sont responsables de la coloration des feuilles, tiges et racines de nombreuses espèces végétales comme par exemple dans le cas de la couleur violette des raisins rouges. On peut citer parmi les anthocyanes : la pélargonidine, la cyanidine, la péonidine, la delphinidine, la pétunidine, la malvidine, l'apigénidine, la lutéolidine, la tricétinidine, la 6-hydroxypélargonidine, la 6-hydroxy cyanidine, la 6-hydroxy delphinidine, l'europinidine, la rosinidine, la capensinidine, la pulchéllidine, l'hirsutidine, la 5-méthylcyanidine, la fisétinidine.

Ces composés anthocyanes peuvent être mis en oeuvre sous forme simple ou sous forme d'hétérosides qui sont la forme la plus fréquemment rencontrée dans la nature.

On peut aussi citer parmi les molécules colorées visibles utilisables dans le procédé de l'invention les mélanines (telles que eumélanine et phéomélanine) par exemple celles présentes dans l'encre de certains céphalopodes comme la seiche, et ses dérivés y compris les analogues synthétiques.

On peut encore citer les acides humiques qui sont des dérivés de polyphénols porteurs de fonctions acide carboxylique ionisables, et ionisées à certains pH. Ce sont des polymères à haut poids moléculaire, chargés négativement, résultant d'un processus de condensation oxydative des composés phénoliques et liés à des acides aminés, à des peptides, et à des des polysaccharides.

Un exemple de structure de type acide humique est illustré ci-dessous :

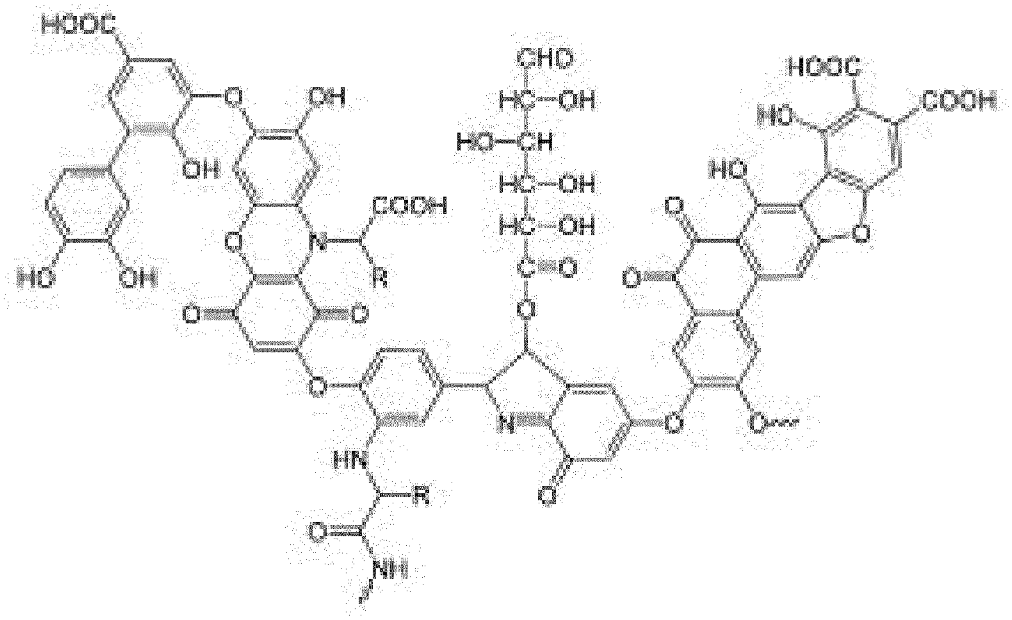

On peut également citer parmi les molécules colorées visibles utilisables dans le procédé de l'invention : le bleu de méthylène ou chlorure de 3,7-bis(diméthylamino)phénothiazine-5-ylium, le cristal violet ou chlorure de tris (4-(diméthylamino)phényl)méthylium, le rouge congo ou acide benzidinediazo-bis-1-naphtylamine-4-sulfonique, les porphyrines, notamment la porphine, l'éosine Y ou acide bromofluorescéique, l'éosine B ou rouge impérial, l'orange thiazole ou *p*-tosylate de 1-méthyl-4-[(3-methyl-2(3*H*)-benzothiazolylidène)méthyl]quinolinium, l'Acid black 1 (CAS 1064-48-8) et l'Acid black 2 (CAS 8005-03-6), le noir ériochrome T ou (4Z)-4-[(1-hydroxynaphthalèn-2-yl- hydrazinylidène]-7-nitro-3-oxo Y-naphtalène-1-sulfonate de sodium, le bleu patenté V (CAS 20262-76-4 et 3536-49-0).

La solution d'au moins une molécule organique chargée colorée utilisable dans le procédé de l'invention peut consister en une solution d'une molécule organique chargée colorée seule, ou d'un mélange de molécules colorées dans un solvant.

Selon une variante de l'invention, on peut prévoir d'utiliser comme solution d'au moins une molécule organique chargée colorée, un extrait, en particulier un extrait aqueux, d'une fraction végétale colorée, comme par exemple un extrait aqueux de fruits tel que le raisin, le sureau, la grenade, l'açaï, un extrait aqueux de pétales de fleurs comme par exemple les pavots, les mauves ou les pivoines, un extrait aqueux de légumes comme la betterave, un extrait aqueux d'une fraction animale colorée, comme par exemple un extrait de cochenille..., un produit dérivé de ces extraits végétaux et animaux, comme par exemple du vin qui est issu du jus de raisin par fermentation alcoolique.

Dans le cas où l'on utilise un extrait animal ou végétal coloré, la totalité des molécules organiques colorées n'est pas nécessairement adsorbée sur le phyllosilicate non gonflant. En effet, seules les molécules chargées s'adsorbent sur le phyllosilicate non gonflant.

On peut également utiliser des mélanges de molécules chargées colorées dans le domaine visible, soit à une longueur d'onde allant de 380 à 780 nm, et de molécules chargées photoluminescentes.

On entend par molécule photoluminescente une molécule qui absorbe dans le domaine ultra-violet, soit à une longueur d'onde allant de 200 à 380 nm, ou dans le visible, soit à une longueur d'onde comprise entre 380 et 780 nm, et qui ré-émet l'énergie absorbée sous forme lumineuse.

Lorsque l'on utilise un mélange de molécules photoluminescentes et de molécules colorées dans le domaine visible, avantageusement, la ou les molécules photoluminescentes sont chargées et une partie de la structure est plane ou sensiblement plane.

Parmi les molécules photoluminescentes utilisables de façon optionnelle dans le procédé de l'invention, on peut citer :

La Rhodamine B ou chlorure de [9-(2-carboxyphényl)-6-diéthylamino-3-xanthénylidène]-diéthylammonium - ou toute autre forme de Rhodamine B comme par exemple le perchlorate de Rhodamine B - , le bromure d'éthydium ou bromure de 3,8-diamino-1-éthyl-6-phénylphénanthridinium, l'iodure de propidium ou di-iodure de 3,8-diamino-5-[3-(diéthylméthylammonio)propyl]-6-phénylphénantridinium, le chlorure de 1,1'-diéthyl-2,2'-cyanine (CAS : 2402-42-8), le iodure de 1,1'-diéthyl-2,2'-dicarbocyanine (CAS : 14187-31-6).

La molécule organique colorée utilisable dans la présente invention est chargée, et la fonction ionique ou ionisable de ladite molécule peut être de nature cationique ou anionique.

Chaque molécule colorée est choisie en fonction du résultat désiré, en termes de composition chimique de la

composition de phyllosilicate colorée et en particulier de la coloration attendue.

La solution (a) d'au moins une molécule organique chargée colorée utilisable dans le procédé de l'invention peut être une solution aqueuse, une solution organique ou une solution hydro-organique. Parmi les solvants organiques utilisables on peut citer le méthanol, l'éthanol, le glycérol, des cétones comme la propanone ou la 2-butanone.

Avantageusement, la solution d'au moins une molécule organique chargée colorée utilisable dans le procédé de l'invention est une solution aqueuse ou une solution hydro-organique comprenant comme solvant un mélange d'eau et d'un ou plusieurs co-solvants tels que des alcools ou des polyols, comme par exemple du méthanol, de l'éthanol, du glycérol, des cétones comme la propanone ou la 2-butanone.

Encore plus avantageusement, la solution d'au moins une molécule organique chargée colorée utilisable dans le procédé de l'invention est une solution aqueuse.

Préparation de la composition de nanoparticules colorées

Le procédé de l'invention comprend la mise en contact, dans un milieu solvant monophasique, d'au moins une molécule organique chargée colorée et de nanoparticules de phyllosilicate non gonflant ayant une épaisseur de 1 nm à 100 nm, et une plus grande dimension de 10 nm à 10 $\mu$m.

Selon l'invention, on entend par « milieu solvant monophasique » un solvant ou un mélange de solvants miscibles entre eux. De tels solvants ne forment qu'une seule phase et ne se séparent pas après être restés sans agitation. La miscibilité est évaluée à la température ambiante.

Avantageusement, on choisit un solvant ou un mélange de solvants dans lesquels la molécule organique chargée colorée est soluble et dans lequel les nanoparticules de phyllosilicate non gonflant sont dispersibles.

Avantageusement, on choisit comme solvant un mélange d'eau et d'un ou plusieurs co-solvants tels que des alcools ou des polyols, comme par exemple du méthanol, de l'éthanol, du glycérol, des cétones comme la propanone ou la 2-butanone.

Encore plus avantageusement, le solvant utilisé dans le procédé de l'invention est de l'eau.

Selon un premier mode de réalisation, les deux composants : la molécule organique chargée colorée et les nanoparticules de phyllosilicate non gonflant, sont introduits sous forme de poudre dans un milieu solvant monophasique et dispersés sous agitation.

Selon un second mode de réalisation, la molécule organique chargée colorée est mise en oeuvre sous forme d'une solution (a) dans au moins un solvant, les nanoparticules de phyllosilicate non gonflant sont introduites sous forme de poudre dans la solution (a). On disperse les nanoparticules de phyllosilicate non gonflant dans la solution (a) sous agitation.

Selon un troisième mode de réalisation, les nanoparticules de phyllosilicate non gonflant sont mises en oeuvre sous forme de dispersion (b) dans au moins un solvant et la molécule organique chargée colorée est introduite sous forme de poudre dans la dispersion (b).

Selon un quatrième mode de réalisation, qui est le mode de réalisation préféré, la molécule organique chargée colorée est fournie sous forme d'une solution (a) dans au moins un solvant, les nanoparticules de phyllosilicate non gonflant sont fournies sous forme d'une suspension (b) dans au moins un solvant, puis la solution (a) et la suspension (b) sont mises en contact.

Dans un procédé selon l'invention, la durée de l'étape (iii) pendant laquelle on met en contact la composition (b) comprenant des particules minérales phyllosilicatées avec la solution (a) comprenant au moins un composé organique colorant chargé, la concentration de chaque composé organique colorant chargé dans la solution colorante (a) et la température à laquelle a lieu cette étape, sont adaptées pour permettre une fixation du composé organique coloré chargé sur les nanoparticules phyllosilicatées et donc une coloration de la composition de nanoparticules phyllosilicatées.

Avantageusement et selon l'invention, la durée pendant laquelle on met en contact ladite composition de nanoparticules phyllosilicatées (b) avec la solution colorante (a) est suffisante pour permettre l'obtention de particules phyllosilicatées colorées. Une durée de quelques secondes peut être suffisante dans certains cas pour obtenir une bonne coloration, notamment à une température suffisante et en présence d'une concentration en composé colorant suffisante et éventuellement en soumettant le mélange comprenant la solution colorante (a) et la composition de nanoparticules phyllosilicatées (b) aux ultrasons. Avantageusement et selon l'invention, la durée pendant laquelle on met en contact la composition de nanoparticules phyllosilicatées (b) avec une solution colorante (a), est supérieure à 2 secondes, notamment comprise entre 2 secondes et 7 jours, en particulier entre 2 secondes et 24 heures, par exemple entre 5 minutes et 1 heure.

Avantageusement, la concentration de la molécule organique colorée chargée présente dans la solution colorante (a) est choisie en tenant compte notamment de la température, de la durée de mise en contact avec la composition de nanoparticules de phyllosilicate (b), de la nature de la composition de nanoparticules de phyllosilicate (b) et de la nature de la ou des molécule(s) organique(s) colorée(s) chargée(s) utilisée(s), ladite concentration étant choisie de façon à être suffisante pour permettre l'obtention de particules minérales phyllosilicatées colorées. Pour la mise en

oeuvre du procédé de l'invention, on utilise avantageusement une solution (a) d'au moins une molécule organique chargée colorée de concentration allant de 0,1 mmol/L à 0,1 mol/L.

L'étape de coloration peut être réalisée à toute température à laquelle la solution colorante est à l'état liquide et permettant l'obtention de particules minérales phyllosilicatées colorées. Avantageusement et selon l'invention, cette étape de coloration a lieu à une température comprise entre 5°C et 100°C. La mise en contact qui a lieu dans cette étape d'un procédé selon l'invention peut par exemple être opérée à température ambiante (20°C à 25°C) ou encore à une température légèrement supérieure à la température ambiante, notamment entre 30°C et 90°C et par exemple entre 40°C et 70°C, en fonction de la nature des molécule(s) organique(s) colorée(s) chargée(s) utilisée(s) et des particules minérales phyllosilicatées à colorer, ainsi que de la nuance et l'intensité de la couleur souhaitée.

L'étape de coloration peut être réalisée en agitant ou non la solution colorante (a) dans laquelle est ajoutée la composition de particules minérales phyllosilicatées (b). Il est par exemple possible de n'agiter que quelques instants manuellement (par exemple à l'aide d'une tige métallique) la solution colorante au moment de l'ajout de la composition de particules minérales phyllosilicatées (b) à la solution colorante (a), puis de la laisser au repos le reste de l'étape de coloration. Avantageusement et selon l'invention, on met en contact ladite composition de particules minérales phyllosilicatées (b) comprenant des particules minérales phyllosilicatées avec la solution colorante (a) sous agitation, par exemple sous agitation magnétique à l'aide d'un agitateur magnétique. Par exemple, une vitesse d'agitation lente est généralement suffisante pour permettre une mise en contact entre la (ou les) molécule(s) organique(s) colorante(s) chargée(s) et la composition de particules minérales phyllosilicatées (b) permettant l'obtention de particules minérales phyllosilicatées colorées.

À l'issue de cette étape (iii) de coloration du procédé selon l'invention, la composition comprenant des particules minérales phyllosilicatées colorées peut être récupérée par élimination de la phase liquide. La phase liquide peut par exemple être éliminée après décantation naturelle de la composition comprenant des particules minérales phyllosilicatées colorées ou encore par centrifugation de la suspension comprenant la composition colorée obtenue. La composition comprenant des particules minérales phyllosilicatées colorées récupérée peut ensuite être rincée de manière à éliminer les colorants ou autres composants non adsorbés sur des particules minérales phyllosilicatées. La composition comprenant des particules minérales phyllosilicatées colorées récupérée peut également être conservée et utilisée sans être rincée. Ainsi, avantageusement, dans un procédé selon l'invention, consécutivement à l'étape de coloration, on rince les particules minérales phyllosilicatées colorées obtenues à l'aide d'une solution organique ou aqueuse dénuée de composé organique coloré chargé.

À l'issue de cette étape (iii) de coloration selon l'invention, la composition comprenant des particules minérales phyllosilicatées colorées peut être conservée ou utilisée en l'état, sous forme de gel ou de suspension aqueuse ou organique ou hydro-organique, ou encore être séchée de manière à éliminer au moins en partie, le solvant, notamment l'eau, encore présent. Avantageusement, on sèche les particules minérales phyllosilicatées colorées obtenues après les étapes (i) à (iii) du procédé de l'invention, et avant ou après un éventuel rinçage. Ce séchage peut être effectué par tout moyen de séchage permettant l'élimination de ce solvant, notamment de cette phase aqueuse. Le séchage peut par exemple être opéré directement dans une étuve (par exemple à une température de l'ordre de 100°C), par atomisation, par séchage par irradiation aux micro-ondes ou encore par lyophilisation. Avantageusement et selon l'invention, on sèche la composition comprenant des particules minérales phyllosilicatées colorées à une température comprise entre 60°C et 200°C.

En particulier, une étape de séchage dans une étuve à une température de l'ordre de 100°C ou 120°C permet d'intensifier ou de nuancer de façon plus ou moins importante, selon la nature du composé organique coloré notamment, la couleur des particules minérales phyllosilicatées colorées.

En outre, il est possible de réitérer au moins une fois l'étape (iii) de coloration au cours de laquelle la composition de particules minérales phyllosilicatées (b) est mise en contact avec la solution colorante (a), soit avec une solution contenant le même composé coloré, soit avec une solution colorante comprenant un composé organique coloré différent. De cette façon, il est possible de modifier ou de nuancer de façon plus ou moins importante la couleur d'une telle composition de manière à obtenir la coloration voulue.

Avantageusement, les nanoparticules hybrides de l'invention présentent un ratio molécule organique colorée/phyllosilicate allant de 0,001 % de carbone à 10 % de carbone, de préférence de 0,01 % de carbone à 5 % de carbone en masse de carbone rapportée à la masse de composé phyllosilicate.

Dans le cas où l'on a employé un composé organique coloré visible, la couleur d'une composition comprenant des particules minérales phyllosilicatées colorées obtenue par un procédé selon l'invention peut être visible à l'oeil nu sous tout type d'éclairage en lumière visible et/ou à l'aide d'un instrument de détection tel qu'un spectrocolorimètre, les particules minérales phyllosilicatées pouvant absorber une partie du spectre visible, et en réfléchir une autre.

Dans le cas où l'on a employé un composé organique coloré photoluminescent, la couleur d'une composition comprenant des particules minérales phyllosilicatées colorées obtenue par un procédé selon l'invention peut être visible à l'oeil nu sous un éclairage en lumière ultra-violette.

Une composition selon l'invention présente une stabilité chimique importante. En outre, de telles compositions

peuvent présenter diverses couleurs, selon la nature chimique des composés organiques colorés fixés aux particules minérales phyllosilicatées. Des compositions comprenant des particules minérales phyllosilicatées colorées, selon l'invention, peuvent être de toutes couleurs, plus ou moins claires ou foncées et plus ou moins intenses.

Extraction de composés colorés

La capacité des nanoparticules de phyllosilicates à fixer de façon irréversible des molécules organiques colorées chargées peut également être utilisée pour extraire d'un milieu des composés colorés, notamment des composés toxiques. Il peut s'agir par exemple, des composés colorés tels que le bromure d'éthydium. Lorsque de tels composés sont dispersés dans un environnement : plan de travail, matériel de laboratoire, peau, muqueuse, il est souhaitable de les collecter en évitant une contamination plus importante. On peut alors utiliser une dispersion aqueuse de nanoparticules de phyllosilicates, que l'on met en contact avec l'environnement contaminé. Les composés organiques chargés ayant une affinité pour les phyllosilicates non gonflants, ceux-ci les fixent de façon irréversible. Le caractère solide des nanoparticules permet une élimination plus facile, plus efficace, dans de meilleures conditions de sécurité, de ces composés organiques.

Les nanoparticules peuvent également être utilisées pour le traitement d'effluents aqueux chargés de colorants, comme par exemple des effluents des industries chimiques, des industries textiles, des industries du cuir.

Dans le cas où un individu ou un animal aurait absorbé un composé organique chargé coloré, notamment toxique, l'absorption par cet individu ou cet animal d'une dispersion aqueuse de nanoparticules de phyllosilicates peut permettre de neutraliser in vivo ces molécules toxiques.

Parmi les molécules organiques chargées concernées par cette application on peut citer notamment le bromure d'éthydium.

**Figures :**

Figure 1 : Diffractogrammes des rayons X de talc nanométrique (----) et d'un produit hybride préparé suivant le protocole de l'exemple 1 (trait continu). L'intensité est représentée en ordonnées en fonction de la distance, exprimée en Angstrom, en abscisses.

Figure 2 : Granulométrie du talc synthétique (échantillon 1 - ■) lors de l'ajout de vin rouge (échantillon 2 - A). La granulométrie (en nm) figure en ordonnées en fonction des numéros d'échantillons en abscisses.

Figure 3 : Spectre RMN $^1H$ du talc synthétique (gris foncé) et de l'hybride "talc synthétique - vin rouge" (gris clair). L'intensité normalisée est représentée en ordonnées. On note les pics d'eau physisorbée à 4,70 ppm et 4,43 ppm, les fonctions silanols à 2,05 ppm et les H structuraux à 0,69 ppm et 0,88 ppm.

Figure 4 : Vue schématique d'un dispositif permettant de mettre en oeuvre un procédé de préparation d'un phyllosilicate de synthèse dans lequel le traitement solvothermal est réalisé en continu.

**Partie expérimentale :**

**I- Matériel et méthodes**

I.A. Matériel

- Cristal violet : disponible commercialement auprès de la société Aldrich sous la référence C6158-50G
- Vert malachite : disponible commercialement auprès de la société Aldrich sous la référence M9015-25G
- Vin rouge : disponible commercialement auprès de la société « Domaine Py » sous la référence « Merlot vieilles vignes », bouteille de 75 cl cuvée 2014.
- Talc naturel : il provient de la carrière Trimouns à Luzenac dans les Pyrénées ariégeoises. Il a été ramassé manuellement, par sélection sur place de la meilleure qualité possible de minerai, du point de vue de la pureté minéralogique (≥ 99 % de talc).
- Talc nanométrique de synthèse : il a été fabriqué suivant le protocole décrit à l'exemple 1 de la demande WO2013/004979.
- Kaolinite nanométrique de synthèse :
  On prépare une solution de nitrate d'aluminium avec 37,51 g (0,1 mole) de nitrate d'aluminium nonahydrate dans 200 mL d'eau pure.

On prépare également une solution de métasilicate de potassium à partir de 29,67 g d'une solution aqueuse de métasilicate de potassium ($K_2SiO_3$) présentant un extrait sec de 52 % (soit 0,1 mole de métasilicate de potassium), de 100 mL de potasse (KOH) à 1 M et de 200 mL d'eau pure.
La première solution de nitrate d'aluminium est ajoutée sous agitation à la solution de métasilicate de potassium et un précipité blanc se forme instantanément.
On agite la suspension obtenue pendant 5 minutes. On réalise ensuite trois cycles de lavage avec de l'eau distillée et de centrifugation à 8000 tr/min pendant 10 minutes à chaque nouvelle centrifugation. Ces lavages successifs avec élimination de la solution surnageante après chaque centrifugation permettent d'éliminer le nitrate de potassium formé au cours de la réaction de précipitation du gel précurseur. On soumet ensuite le gel précurseur placé dans un réacteur en titane clos disposé dans un four à un traitement hydrothermal à une température de 300°C pendant 24 heures sous la pression de vapeur saturante de l'eau dans le réacteur. Après refroidissement jusqu'à la température ambiante, le réacteur est ouvert et la suspension obtenue est centrifugée. Après centrifugation, on récupère une composition comprenant des particules de composé de formule $Al_2Si_2O_5(OH)_4$. La composition de particules récupérées après centrifugation est séchée à l'étuve (120°C, 12 heures) puis broyée au mortier. La composition obtenue se présente sous la forme d'une poudre de couleur blanche.

Le diffractogramme des rayons X de cette composition présente les raies de diffraction caractéristiques suivantes :

- un plan (001) situé à une distance de 7,15 Å ;
- un plan (020) situé à une distance de 4,46 Å ;
- un plan (110) situé à une distance de 4,37 Å ;
- un plan (111) situé à une distance de 4,16 Å ;
- un plan (021) situé à une distance de 3,80 Å ;
- un plan (002) situé à une distance de 3,56 Å ;
- un plan (130) et un plan (201) situés à une distance de 2,56 Å ;
- un plan (131) et un plan (200) situés à une distance de 2,50 Å ;
- un plan (202) et un plan (131) situés à une distance de 2,33 Å ;
- un plan (060), un plan (331) et un plan (331) situés à une distance de 1,49 Å.

Le spectre en moyen infrarouge de la composition de kaolinite synthétique obtenue présente quatre bandes de vibration à 3620 $cm^{-1}$, à 3651 $cm^{-1}$, à 3667 $cm^{-1}$ et à 3693 $cm^{-1}$ représentatives des vibrations d'élongation des groupements hydroxyle (-OH) de la kaolinite synthétique.

- Mica nanométrique de synthèse :

On prépare 300 mL d'une solution aqueuse de sulfate de magnésium (33,27 g soit 0,135 mole) et d'acide sulfurique (120 g d'une solution à 0,5 M).

On prépare ensuite une solution de métasilicate de potassium en diluant 59,35 g (soit 0,2 mole) d'une solution aqueuse de métasilicate de potassium ($K_2SiO_3$) à 52 % d'extrait sec dans 150 mL d'eau déminéralisée. Cette solution de métasilicate de potassium est ajoutée à la solution précédente et un précipité blanc se forme instantanément.

On agite la suspension obtenue pendant 5 minutes. On réalise ensuite trois cycles de lavage avec de l'eau distillée et de centrifugation à 8000 tours/min pendant 10 minutes à chaque nouvelle centrifugation. Ces lavages successifs avec élimination de la solution surnageante après chaque centrifugation permettent d'éliminer le sulfate de potassium formé au cours de la réaction de précipitation du gel précurseur. Enfin, le précipité blanc récupéré est mis en suspension dans de l'eau déminéralisée jusqu'à un volume final de 500 ml et soumis aux ultrasons sous agitation magnétique pendant 10 minutes jusqu'à obtention d'une suspension homogène, de couleur blanche, de gel précurseur.

On ajoute ensuite au gel précurseur 988 mg de potasse KOH hydratée (contenant 85 % de potasse et 15 % d'eau, soit 0,015 mole de potasse pure ajoutée) préalablement diluée dans 30 mL d'eau déminéralisée, et on agite magnétiquement la suspension obtenue pendant 5 minutes à la température ambiante (22,5°C).

On soumet ensuite le gel précurseur placé dans un réacteur en titane clos disposé dans un four à un traitement hydrothermal à une température de 300°C pendant 24 heures sous la pression de vapeur saturante de l'eau dans le réacteur.

Après refroidissement jusqu'à la température ambiante, le réacteur est ouvert et la suspension obtenue est centrifugée. Après centrifugation, on récupère une composition comprenant au moins 80 % en poids de particules de composé de formule $K_{0,3}\,Si_4\,Mg_{2,7}\,O_{10}\,(OH)_2$.

La composition de particules récupérées après centrifugation est séchée à l'étuve pendant 12 heures à 120°C puis broyée dans un mortier. La composition obtenue se présente sous la forme d'une poudre de couleur blanche.

Le diffractogramme des rayons X de la composition de particules de composé de formule $K_{0,3}$ $Si_4$ $Mg_{2,7}$ $O_{10}$ $(OH)_2$, ainsi obtenue présente les raies de diffraction caractéristiques suivantes :

- un plan (001) situé à une distance de 10,15 Å ;
- un plan (002) situé à une distance de 5,03 Å ;
- un plan (020) situé à une distance de 4,53 Å ;
- des plans (003) et (022) situés à une distance de 3,34 Å ;
- un plan (131) situé à une distance de 2,60 Å ;
- un plan (005) situé à une distance de 2,01 Å ;
- un plan (060) situé à une distance de 1,52 Å.

On soumet ensuite la composition à un traitement thermique anhydre à 550°C dans un four pendant 5 heures. La composition obtenue après le traitement thermique anhydre reste blanche.

Le diffractogramme des rayons X de la composition de particules de composé de formule $K_{0,3}$ $Si_4$ $Mg_{2,7}$ $O_{10}$ $(OH)_2$ obtenue après un traitement thermique anhydre à 550°C présente, après le traitement thermique anhydre, les raies de diffraction caractéristiques suivantes :

- un plan (001) situé à une distance de 10,24 Å ;
- un plan (002) situé à une distance de 5,02 Å ;
- un plan (020) situé à une distance de 4,56 Å ;
- des plans (003) et (022) situés à une distance de 3,37 Å ;
- un plan (131) situé à une distance de 2,60 Å ;
- un plan (005) situé à une distance de 2,02 Å ;
- un plan (060) situé à une distance de 1,52 Å.

I.B. Méthodes

I.B.1. Synthèses

Exemple 1 : Synthèse de la composition de nanomatériau hybride à base de talc de synthèse et de vin rouge :

40 millilitres de vin rouge sont ajoutés à température ambiante à une suspension de 1 g de nanotalc dans 100 mL d'eau pure. La suspension rose est agitée sous sonication pendant 5 minutes puis centrifugée à 14000 tr/min pendant 20 min. On note que le surnageant est légèrement coloré ce qui traduit l'adsorption partielle des colorants du vin sur le minéral. Le culot est constitué d'une pâte de couleur rose. Cette pâte peut être séchée sous étuve (60°C pendant 12h) ou par lyophylisation ou toute autre technique de séchage.

Exemple 2 : Synthèse de la composition de nanomatériau hybride à base de kaolinite de synthèse et de vin rouge :

On a appliqué le même protocole qu'à l'exemple 1, en utilisant la kaolinite de synthèse dont la préparation est détaillée ci-dessus.

Exemple 3 : Synthèse de la composition de nanomatériau hybride à base de mica de synthèse et de vin rouge :

On a appliqué le même protocole qu'à l'exemple 1, en utilisant le mica de synthèse dont la préparation est détaillée ci-dessus.

I.B.2. Tests

Caractérisation - test n°1 : Test d'adsorption :

a) Protocole général

Afin de mettre en évidence le fort pouvoir adsorbant des minéraux de synthèse ainsi que le mode d'interaction des molécules organiques avec le talc, différents tests d'adsorption ont été réalisés. Dans un bécher, on mélange un minéral sous forme de suspension aqueuse avec un colorant dissous dans un solvant. Si nécessaire, on ajoute de l'eau pour que le mélange soit suffisamment liquide. Le tout est ensuite été agité et passé sous ultrasons jusqu'à ce que le mélange soit homogène et sans agglomérat visible. L'ultime étape consiste à centrifuger le mélange à 9000 tours/min

pendant environ 20 minutes. A l'issue de cela, le minéral se retrouve plaqué au fond du pot surmonté du surnageant. De ce fait, si le minéral a totalement adsorbé le colorant, il se trouve coloré au fond du pot et le surnageant est incolore et limpide. Au contraire, s'il n'a pas adsorbé le colorant, le minéral garde sa couleur initiale et le surnageant est coloré. Si à la fin de cette première centrifugation le surnageant est coloré et que le minéral l'est aussi, cela signifie que le minéral n'a pas adsorbé la totalité du colorant. Le surnageant est alors remplacé par de l'eau désionisée dans le pot pour une seconde centrifugation. Cette opération permet de voir si l'adsorption est forte ou si le minéral rejette le colorant (leaching). Cette étape de remise en centrifugation est réalisée autant de fois que nécessaire.

b) Test de comparaison du pouvoir adsorbant du talc naturel et synthétique

Tout d'abord, des tests d'adsorption ont été réalisés pour comparer le pouvoir adsorbant du talc de synthèse avec celui du talc naturel. Pour cela, différents colorants organiques ont été choisi, à savoir le cristal violet, le vert malachite, le vin rouge. Ces derniers ont été mis en contact avec du talc synthétique ou du talc naturel dans un même rapport « quantité de colorant/quantité de minéral » pour une meilleure comparaison des résultats. A l'issue de la centrifugation, la couleur des minéraux et du surnageant sont comparées pour évaluer le pouvoir adsorbant du minéral.

Ces colorants ont été choisis car ils présentent tous une charge positive ou négative en surface.

Différents tests ayant été réalisés avec des colorants non chargés (β-carotène, curcumine, non rapportés ici) ont montrés des résultats négatifs dans le test d'adsorption sur le talc de synthèse.

Caractérisation - Tests N°2 : Méthodes de caractérisation

a) Diffraction des rayons X

Les analyses DRX ont été réalisées sur un appareil de type D2 Phaser de la société Bruker, avec une longueur d'onde de 1,54060 ($\lambda_{Cu}$), sur un domaine d'angle de diffraction allant de 0 à 80 °2θ. Par ailleurs, l'étude des diffractogrammes a permis de déterminer le domaine de cohérence du minéral, c'est-à-dire le nombre de feuillets TOT empilés selon l'axe c* sans défaut majeur. Ce domaine de cohérence a été calculé grâce à la formule de Scherrer : $L = \frac{0,91 \, x \, \lambda}{B \, x \cos(\theta)}$ où L correspond à la taille du domaine cohérent (Å), λ à la longueur d'onde du rayonnement, B à la largeur du pic à mi-hauteur (rad) et θ l'angle de la raie de diffraction.

b) Granulométrie

Les mesures de granulométrie du talc synthétique ont été réalisées avec un granulomètre Vasco-2 de Cordouan Technologies, spécifique pour la détection de nanoparticules. Les résultats d'analyse correspondent à une étude statistique comprenant 20 mesures avec un temps d'acquisition d'une minute par mesure.

c) Résonance Magnétique Nucléaire du $^1H$

Une analyse RMN $^1H$ a été réalisée au moyen d'un spectromètre Bruker Avance 400 équipé d'une sonde 4 mm opérant à 399,60 MHz pour le proton $^1H$.

f) Analyseur de carbone

Enfin, un analyseur de carbone a été utilisé afin de mesurer la quantité de carbone qui s'est adsorbée sur les nanoparticules de talc synthétique lors des tests d'adsorption (pour un même rapport « quantité de colorant/quantité de minéral »). Les mesures ont été réalisées sur un appareil HORIBA - Carbon/Sulfur analyzer du modèle EMIA - 320V. L'échantillon est disposé dans un creuset qui est mis à chauffer pendant 110 secondes dans un four avec une rampe de température de 0 à 1500°C. Lors de ce chauffage, l'échantillon est vaporisé et se transforme en gaz (dont le $CO_2$). Un détecteur infrarouge mesure alors le pourcentage de carbone provenant de l'échantillon. Lors de la manipulation, le standard NCS HC16024 contenant 3,59 % de C a été passé en entrée et en sortie d'analyse afin de valider les résultats.

I.B. Résultats

I.B.1. Résultats des tests d'adsorption

a) Pouvoir adsorbant du talc de synthèse

Les résultats des tests d'adsorption du talc naturel et du talc synthétique sont les suivants : Dans tous les cas, on remarque qu'après le passage à la centrifugeuse, le mélange qui était homogène et coloré dans son ensemble présente deux phases distinctes : un précipité de talc coloré au fond du pot et un surnageant plus ou moins coloré. Dans le cas du talc naturel, le surnageant est à chaque fois un peu coloré ce qui signifie que le minéral n'a pas totalement adsorbé le colorant. Au contraire, dans le cas du talc synthétique, le surnageant est à chaque fois parfaitement incolore et limpide ce qui signifie que le minéral a complètement adsorbé le colorant. Une exception provient du test au vin où le talc synthétique n'adsorbe pas l'intégralité des composés mais seulement une partie des colorants du vin.

I.B.2. Caractérisation de l'interaction « Talc synthétique - vin rouge »

a) DRX

En présence de vin rouge, la raie (001) du talc se décale vers les petits angles, marquant ainsi une augmentation de la distance inter-réticulaire entre les feuillets (figure 1). Il semblerait donc que les colorants rouges du vin (anthocyanes) s'adsorbent également sur les surfaces basales du talc.

b) Granulométrie

Suite à l'adsorption des anthocyanes du vin rouge, les deux populations de particules de talc qui avaient une taille moyenne de 45 nm et 259 nm voient leur taille augmenter à 65 nm et 543 nm (figure 2) du fait d'une probable agglomération des particules due à l'interaction des anthocyanes entre-elles.

c) RMN

Les résultats RMN du $^1H$ sur l'hybride « talc synthétique - vin rouge » (figure 3) montrent qu'en présence d'anthocyanes dans le système, le spectre RMN ne présente pas le pic caractéristique des fonctions silanols Si - OH centré à 2,05 ppm. Cela indiquerait que le colorant a interagi avec ces fonctions ce qui a modifié l'environnement chimique autour de l'atome d'hydrogène.

d) Analyse du Carbone

Enfin, une analyse de carbone a été réalisée afin de comparer la quantité de colorant qui a été adsorbée entre le talc synthétique et le talc naturel lors des tests d'adsorption. Les résultats d'analyse ont été validés par le standard NCS HC16024 contenant 3,59 %. Le tableau 1 ci-dessous montre les résultats obtenus.

Tableau 1

| | Talc naturel + vin | Talc synthétique + vin | NCS HC16024 « entrée » | NCS HC16024 « sortie » |
|---|---|---|---|---|
| % C | 0,8304 | 4,3028 | 3,61 | 3,55 |

Ces résultats montrent que le talc synthétique a adsorbé environ 5 fois plus de colorants que le talc naturel. Cette analyse met donc bien en avant le fort pouvoir d'adsorption du minéral de synthèse.

**Revendications**

1. Procédé de préparation d'une composition hybride organique/inorganique comprenant des nanoparticules minérales fonctionnalisées par au moins une molécule choisie parmi les molécules organiques chargées colorées, ce procédé comprenant au moins la mise en contact, dans un milieu solvant monophasique, d'au moins une molécule organique chargée colorée et de nanoparticules de phyllosilicate non gonflant ayant une épaisseur de 1 nm à 100 nm, et une plus grande dimension de 10 nm à 10 μm.

2. Procédé selon la revendication 1, qui comprend au moins les étapes suivantes :

(i) Fourniture d'une solution (a) d'au moins une molécule organique chargée colorée dans au moins un solvant,
(ii) Fourniture d'une suspension (b) de nanoparticules de phyllosilicate non gonflant dans au moins un solvant,
(iii) Mise en contact de la solution (a) et de la suspension (b),

les nanoparticules de phyllosilicate non gonflant ayant une épaisseur de 1 nm à 100 nm, et une plus grande dimension de 10 nm à 10 $\mu$m.

3. Procédé selon la revendication 1 ou selon la revendication 2, qui comprend en outre les étapes suivantes :

- Elimination de la phase solvant,
- Récupération des nanoparticules.

4. Procédé selon l'une quelconque des revendications précédentes, dans lequel les phyllosilicates non gonflants répondent à l'une des formules chimiques suivantes :

■ $(SixGe(1-x))_4M_3O_{10}(OH)_2$, (I) dans laquelle :

- x est un nombre réel de l'intervalle [0 ; 1],
- M désigne au moins un métal divalent ayant pour formule $Mg_{y1}Co_{y2}\,Zn_{y3}Cu_{y4}Mn_{y5}Fe_{y6}Ni_{y7}Cr_{y8}$; chaque indice yi représentant un nombre réel de l'intervalle [0 ; 1], et tel que $\sum_{i=1}^{8} yi = 1$ ,
ou

■ $(Aly'M'(1-y'))_2(Six'Ge(1-x'))_2O_5(OH)_4$, (II) dans laquelle :

- M' désigne au moins un métal trivalent choisi dans le groupe formé du gallium et des terres rares,
- y' est un nombre réel de l'intervalle [0 ; 1],
- x' est un nombre réel de l'intervalle [0 ; 1],
ou

■ $At(Six''Ge(1-x''))_4M''_kO_{10}(OH)_2$, (III) dans laquelle :

- A désigne au moins un cation monovalent d'un élément métallique ayant pour formule $Li_{w1}Na_{w2}K_{w3}Rb_{w4}Cs_{w5}$, chaque wi représentant un nombre réel de l'intervalle [0 ;1] tel que $\sum_{i=1}^{5} wi = 1$ .
- x" est un nombre réel de l'intervalle [0 ; 1],
- M" désigne au moins un métal divalent ayant pour formule $Mg_{j1}Co_{j2}\,Zn_{j3}Cu_{j4}Mn_{j5}Fe_{j6}Ni_{j7}Cr_{j8}$ ; chaque indice ji représentant un nombre réel de l'intervalle [0 ; 1], et tel que $\sum_{i=1}^{8} ji = 1$ ,
- k est un nombre réel de l'intervalle [2,50 ; 2,85],
- t+2k est un nombre réel de l'intervalle [5,3 ; 6,0]

5. Procédé selon la revendication 4, dans lequel les phyllosilicates non gonflants sont formés d'un empilement de feuillets élémentaires :

■ de type phyllosilicate 2:1 et de formule chimique $Si_4M_3O_{10}(OH)_2$, plus particulièrement de formule chimique $Si_4Mg_3O_{10}(OH)_2$,
ou
■ de type phyllosilicate 1:1 et de formule chimique $Al_2Si_2O_5(OH)_4$,
ou
■ de type phyllosilicate 2:1 et de formule chimique $K\,Si_4\,Mg_{2,5}\,O_{10}\,(OH)_2$ (IIId) ou $K_{0,8}\,Si_4\,Mg_{2,6}\,O_{10}\,(OH)_2$ (IIIf).

6. Procédé selon l'une quelconque des revendications précédentes dans lequel la molécule organique colorée est choisie parmi :

- les polyphénols, en particulier les anthocyanes, tels que la pélargonidine, la cyanidine, la péonidine, la delphinidine, la pétunidine, la malvidine, l'apigénidine, la lutéolidine, la tricétinidine, la 6-hydroxypélargonidine, la 6-hydroxy cyanidine, la 6-hydroxy delphinidine, l'europinidine, la rosinidine, la capensinidine, la pulchéllidine, l'hirsutidine, la 5-méthylcyanidine, la fisétinidine, les mélanines telles que eumélanine et phéomélanine et leurs dérivés, y compris synthétiques, les acides humiques,
- le bleu de méthylène ou chlorure de 3,7-bis(diméthylamino)phénothiazine-5-ylium, le cristal violet ou chlorure de tris (4-(diméthylamino)phényl)méthylium, le rouge congo ou acide benzidinediazo-bis-1-naphtylamine-4-sulfonique, les porphyrines, notamment la porphine, l'éosine Y ou acide bromofluorescéique, l'éosine B ou rouge impérial, l'orange thiazole ou p-tosylate de 1-méthyl-4-[(3-methyl-2(3*H*)-benzothiazolylidène)méthyl]quinolinium, l'Acid black 1 et l'Acid black 2, le noir ériochrome T ou (4Z)-4-[(1-hydroxynaphthalén-2-yl- hydrazinylidène]-7-nitro-3-oxo Y-naphtalène-1-sulfonate de sodium, le bleu patenté V,
- un extrait aqueux d'une fraction végétale colorée, comme par exemple un extrait aqueux de raisin, de sureau, de grenade, d'açaï, de pétales de pavots, de mauves, de pivoines ou de betterave,
- un extrait aqueux d'une fraction animale colorée, comme par exemple un extrait de cochenille,
- un produit dérivé de ces extraits végétaux et animaux, comme par exemple du vin,
- les mélanges de ces composés

**7.** Composition de nanoparticules hybrides organique/inorganique comprenant au moins un phyllosilicate non gonflant ayant une épaisseur de 1 nm à 100 nm, et une plus grande dimension de 10 nm à 10 μm ; et au moins une molécule choisie parmi les molécules organiques chargées colorées, ladite molécule organique étant adsorbée sur le phyllosilicate, cette composition étant susceptible d'être obtenue par le procédé selon l'une quelconque des revendications 1 à 6.

**8.** Composition selon la revendication 7, dans laquelle le ratio molécule organique colorée/phyllosilicate est de 0.001 % de carbone à 10 % de carbone, de préférence de 0.01 % de carbone à 5 % de carbone, en masse de carbone rapportée à la masse de phyllosilicate.

**9.** Utilisation de nanoparticules de phyllosilicate non gonflant, ayant une épaisseur de 1 nm à 100 nm, et une plus grande dimension de 10 nm à 10 μm, pour extraire d'un environnement une molécule organique chargée colorée, selon laquelle lesdites nanoparticules de phyllosilicate non gonflant forment, par adsorption de ladite molécule organique chargée colorée, une composition hybride organique/inorganique, étant entendu que lorsque ledit environnement est un tissu biologique, il s'agit d'un tissu biologique isolé ou cultivé.

## Patentansprüche

**1.** Verfahren zur Herstellung einer hybriden organischen/anorganischen Zusammensetzung, umfassend anorganische Nanopartikel, die mit mindestens einem Molekül funktionalisiert sind, das ausgewählt ist aus den gefärbten geladenen organischen Molekülen, dieses Verfahren umfassend mindestens das Inkontaktbringen, in einem einphasigen Lösungsmittelmedium, mindestens eines gefärbten geladenen organischen Moleküls und von nicht quellenden Phyllosilikat-Nanopartikeln, die eine Stärke von 1 nm bis 100 nm und eine größte Abmessung von 10 nm bis 10 μm aufweist.

**2.** Verfahren nach Anspruch 1, das mindestens die folgenden Schritte umfasst:

(i) Bereitstellen einer Lösung (a) aus mindestens einem gefärbten geladenen organischen Molekül in einem Lösungsmittel,
(ii) Bereitstellen einer Suspension (b) von nicht quellenden Phyllosilikat-Nanopartikeln in mindestens einem Lösungsmittel,
(iii) Inkontaktbringen der Lösung (a) und der Suspension (b),

wobei die nicht quellenden Phyllosilikat-Nanopartikel eine Stärke von 1 nm bis 100 nm und eine größte Abmessung von 10 nm bis 10 μm aufweisen.

**3.** Verfahren nach Anspruch 1 oder nach Anspruch 2, das ferner die folgenden Schritte umfasst:

- Eliminieren der Lösungsmittelphase,
- Rückgewinnen von Nanopartikeln.

**4.** Verfahren nach einem der vorherigen Ansprüche, wobei die nicht quellenden Phyllosilikate einer der folgenden chemischen Formeln entsprechen:

■ $(SixGe(1\text{-}x))_aM_3O_{10}(OH)_2$, (I) wobei:

- x eine reelle Zahl aus dem Intervall [0; 1] ist,
- M mindestens ein zweiwertiges Metall bezeichnet, das als Formel $Mg_{y1}Co_{y2}\ Zn_{y3}Cu_{y4}Mn_{y5}Fe_{y6}Ni_{y7}Cr_{y8}$ aufweist, wobei jeder Index yi eine reelle Zahl aus dem Intervall [0; 1] darstellt, und sodass $\sum_{i=1}^{8} yi = 1$, oder

■ $(Aly'M'(1\text{-}y'))_2(Six'Ge(1\text{-}x'))_2O_5(OH)_4$, (II) wobei:

- M' mindestens ein dreiwertiges Metall bezeichnet, das ausgewählt ist aus der Gruppe, bestehend aus Gallium und seltenen Erden,
- y' eine reelle Zahl aus dem Intervall [0; 1] ist,
- x' eine reelle Zahl aus dem Intervall [0; 1] ist,
oder

■ $At(Six''Ge(1\text{-}x''))_4M''_kO_{10}(OH)_2$, (III) wobei:

- A mindestens ein einwertiges Kation eines Metallelements bezeichnet, das die Formel $Li_{w1}Na_{w2}Kw_3Rb_{w4}Cs_{w5}$ aufweist, wobei jedes wi eine reelle Zahl aus dem Intervall [0; 1] darstellt, soass $\sum_{i=1}^{5} wi = 1$
- x" eine reelle Zahl aus dem Intervall [0; 1] ist,
- M" mindestens ein zweiwertiges Metall bezeichnet, das als Formel $Mg_{j1}Co_{j2}\ Zn_{j3}Cu_{j4}Mn_{j5}Fe_{j6}Nij7Cr_{j8}$ aufweist, wobei jeder Index ji eine reelle Zahl aus dem Intervall [0; 1] darstellt, und sodass $\sum_{i=1}^{8} ji = 1$,
- k eine reelle Zahl aus dem Intervall [2,50; 2,85] ist,
- t+2k eine reelle Zahl aus dem Intervall [5,3; 6,0] ist.

**5.** Verfahren nach Anspruch 4, wobei die nicht quellenden Phyllosilikate aus einem Stapel von Elementarblättchen gebildet sind:

■ vom Typ Phyllosilikat 2:1 und der chemischen Formel $Si_4M_3O_{10}(OH)_2$, insbesondere der chemischen Formel $Si_4Mg_3O_{10}\ (OH)_2$,
oder
■ vom Typ Phyllosilikat 1:1 und der chemischen Formel $Al_2Si_2O_5(OH)_4$,
oder
■ vom Typ Phyllosilikat 2:1 und der chemischen Formel $K\ Si_4\ Mg_{2,5}\ O_{10}\ (OH)_2$ (IIId) ou $K_{0,8}\ S_{14}\ Mg_{2,6}O_{10}\ (OH)_2$ (IIIf).

**6.** Verfahren nach einem der vorherigen Ansprüche, wobei das gefärbte organische Molekül ausgewählt ist aus:

• Polyphenolen, insbesondere Anthocyanen, wie z. B. Pelargonidin, Cyanidin, Peonidin, Delphinidin, Petunidin, Malvidin, Apigenidin, Luteolidin, Trietinidin, 6-Hydroxypelargonidin, 6-Hydroxycyanidin, 6-Hydroxydelphinidin, Europinidin, Rosinidin, Capensidin, Pulchellidin, Hirsutidin, 5-Methylcyanidin, Fisetinidin, Melanine wie Eumelanin und Phäomelanin und ihrer Derivate, einschließlich synthetische, Huminsäuren,
• Methylenblau oder 3,7-Bis(dimethylamino)phenothiazin-5-yliumchlorid, violetter Kristall oder tris(4-(Dimethylamino)phenyl)methyliumchlorid, Kongorot oder Benzidinediazo-bis-1-naphthylamin-4-sulfonsäure, Porphyrine, insbesondere Porphin, Eosin Y oder Bromfluoreszensäure, Eosin B oder Imperialrot, orangefarbenes Thiazol oder *p*-Tosylat von I-Methyl-4-[(3-methyl-2(3*H*)-benzothiazolyliden)methyl]chinolinium, Säureschwarz 1 und Säureschwarz 2, schwarzes Eriochrom T oder (4Z)-4-[(1-Hydroxynaphthalin-2-ylhydrazinyliden]-7-nitro-3-oxo Y-naphthalin-1-sulfonsäurenatrium, Patentblau V,
• einem wässrigen Extrakt einer gefärbten pflanzlichen Fraktion, wie z. B. ein wässriger Extrakt aus Trauben, Holunder, Granatapfel, Acai, Mohnblüten, Malven, Pfingstrosen oder Rote Bete,

• einem wässrigen Extrakt einer gefärbten tierischen Fraktion, wie z. B. ein Wollläuseextrakt,
• einem aus diesen pflanzlichen und tierischen Extrakten gewonnenen Erzeugnis, wie z. B. Wein,
• Gemischen dieser Verbindungen.

**7.** Hybride organische/anorganische Nanopartikelzusammensetzung, umfassend mindestens ein nicht quellendes Phyllosilikat, das eine Stärke von 1 nm bis 100 nm und eine größte Abmessung von 10 nm bis 10 μm aufweist; und mindestens ein Molekül, ausgewählt aus gefärbten geladenen organischen Molekülen, wobei das organische Molekül an das Phyllosilikat adsorbiert ist, wobei diese Zusammensetzung durch das Verfahren gemäß einem der Ansprüche 1 bis 6 erlangt werden kann.

**8.** Zusammensetzung nach Anspruch 7, wobei das Verhältnis von gefärbtem organischem Molekül zu Phyllosilikat 0,001 % Kohlenstoff bis 10 % Kohlenstoff, vorzugsweise 0,01 % Kohlenstoff bis 5 % Kohlenstoff, bezogen auf die Masse des Kohlenstoffs, bezogen auf die Masse des Phyllosilikats, ist.

**9.** Verwendung von nicht quellenden Phyllosilikat-Nanopartikeln, die eine Stärke von 1 nm bis 100 nm und eine größere Abmessung von 10 nm bis 10 μm aufweisen, zum Extrahieren eines gefärbten geladenen organischen Moleküls aus einer Umgebung, wobei die nicht quellenden Phyllosilikat-Nanopartikel durch Adsorption des gefärbten geladenen organischen Moleküls eine hybride organische/anorganische Zusammensetzung bilden, mit der Maßgabe, dass, wenn die Umgebung ein biologisches Gewebe ist, es ein isoliertes oder gezüchtetes biologisches Gewebe ist.

## Claims

**1.** Method for preparing an organic/inorganic hybrid composition comprising mineral nanoparticles functionalized by at least one molecule chosen from colored charged organic molecules, this method comprising at least bringing into contact, in a monophasic solvent medium, with at least one colored charged organic molecule and non-swelling phyllosilicate nanoparticles having a thickness of 1 nm to 100 nm, and a larger dimension of 10 nm to 10 μm.

**2.** Method according to claim 1, which comprises at least the following steps:

(i) providing a solution (a) of at least one colored charged organic molecule in at least one solvent,
(ii) providing a suspension (b) of non-swelling phyllosilicate nanoparticles in at least one solvent,
(iii) contacting the solution (a) and the suspension (b), the non-swelling phyllosilicate nanoparticles having a thickness of 1 nm to 100 nm, and a larger dimension of 10 nm to 10 μm.

**3.** Method according to claim 1 or claim 2, which further comprises the following steps:

- Elimination of the solvent phase,
- Recovery of nanoparticles.

**4.** Method according to any one of the preceding claims wherein the non-swelling phyllosilicates have one of the following chemical formulas:

• $(SixGe(1\text{-}x))_4M_3O_{10}(OH)_2$, (I) in which:

- x is a real number of the interval [0; 1],
- M denotes at least one divalent metal having the formula $Mg_{y1}Co_{y2}Zn_{y3}Cu_{y4}Mn_{y5}Fe_{y6}Ni_{y7}Cr_{y8}$; each index yi representing a real number of the interval [0; 1], and such that $\sum_{i=1}^{8} yi = 1$ ,
or

• $(Aly'M'(1\text{-}y'))_2(Six'Ge(1\text{-}x'))_2O_5(OH)_4$, (II) in which:

- M' denotes at least one trivalent metal chosen from the group formed of gallium
- and rare earths,
- y' is a real number of the interval [0; 1],
- x' is a real number of the interval [0; 1],

or

- $A_t(Si_{x''}Ge_{(1-x'')})_4M''_kO_{10}(OH)_2$, (III) in which:

- A denotes at least one monovalent cation of a metal element having the formula $Li_{w1}Na_{w2}K_{w3}Rb_{w4}Cs_{w5}$, each wi representing a real number of the interval [0; 1] such that $\sum_{i=1}^{5} wi = 1$.
- x" is a real number of the interval [0; 1],

- M" denotes at least one divalent metal having the formula $Mg_{j1}Co_{j2}Zn_{j3}Cu_{j4}Mn_{j5}Fe_{j6}Ni_{j7}Cr_{j8}$; each index ji representing a real number of the interval [0; 1], and such that $\sum_{i=1}^{8} ji = 1$,

- k is a real number in the range [2.50; 2.85]
- t+2k is a real number of the interval [5.3; 6.0]

**5.** Method according to claim 4, in which the non-swelling phyllosilicates are formed of a stack of elementary sheets:

- of 2:1 type phyllosilicate and of chemical formula $Si_4M_3O_{10}(OH)_2$, more particularly of chemical formula $Si_4M_3O_{10}(OH)_2$,

or

- of 1:1 type phyllosilicate and of chemical formula $Al_2Si_2O_5(OH)_4$.

or

- of 2:1 type phyllosilicate and of chemical formula K $Si_4Mg_{2,5}O_{10}(OH)_2$(IIId) or $K_{0,8}$ $Si_4Mg_{2,6}O_{10}(OH)_2$(IIIf).

**6.** Method according to any one of the preceding claims, in which the colored organic molecule is chosen from:

- polyphenols, in particular anthocyanins, such as pelargonidine, cyanidine, peonidine, delphinidin, petunidine, malvidine, apigenidine, luteolidine, tricetinidine, 6-hydroxypelargonidine, 6-hydroxy cyanidine, 6-hydroxy delphinidin, europinidine, rosinidine, capensinidine, pulchelidine, hirsutidine, 5-methylcyanidine, fisetinidine, melanins such as eumelanin and pheomelanin and their derivatives, including synthetic, humic acids,
- methylene blue or 3,7-bis(dimethylamino)phenothiazine-5-ylium chloride, crystal violet or tris(4-(dimethylamino) phenyl)methylium chloride, congo red or benzidinediazo-bis-1-naphthylamine-4-sulfonic, porphyrins, especially porphine, eosin $\gamma$ or bromofluorescent acid, eosin B or imperial red, thiazole orange or p-tosylate of 1-methyl-4-[(3-methyl-2)(3H)benzothiazolylidene)methyl] quinolinium, acid black 1 and acid black 2, eriochrome black T or (4Z)-4-[(1-hydroxynaphthalen-2-yl-hydrazinylidene]-7-nitro-3-oxo-naphthalene-1-sulphonate sodium, patent blue V,
- an aqueous extract of a colored vegetable fraction, such as an aqueous extract of grapes, elderberries, pomegranates, acai, poppy petals, mauves, peonies or beetroot,
- an aqueous extract of a colored animal fraction, for example a cochineal extract,
- a product derived from these plant and animal extracts, such as wine,
- mixtures of these compounds

**7.** Hybrid organic/inorganic nanoparticle composition comprising at least one non-swelling phyllosilicate having a thickness of 1 nm to 100 nm, and a larger dimension of 10 nm to 10 $\mu$m and at least one molecule chosen from colored charged organic molecules, said organic molecule being adsorbed on the phyllosilicate, this composition being obtainable by the method according to one of the any of claims 1 to 6.

**8.** Composition according to claim 7, wherein the ratio of organic colored molecule/phyllosilicate is from 0.001% of carbon to 10% of carbon, preferably from 0.01% of carbon to 5% of carbon, mass of carbon relative to the mass of phyllosilicate.

**9.** Use of non-swelling phyllosilicate nanoparticles, having a thickness of 1 nm to 100 nm, and a larger dimension of 10 nm to 10 $\mu$m, for extracting from an environment a colored organic charged molecule, wherein said non-swelling phyllosilicate nanoparticles form, by adsorption of said colored organic charged molecule, an organic/inorganic hybrid composition, it being understood that when said environment is a biological tissue, it is an isolated or cultivated biological tissue.

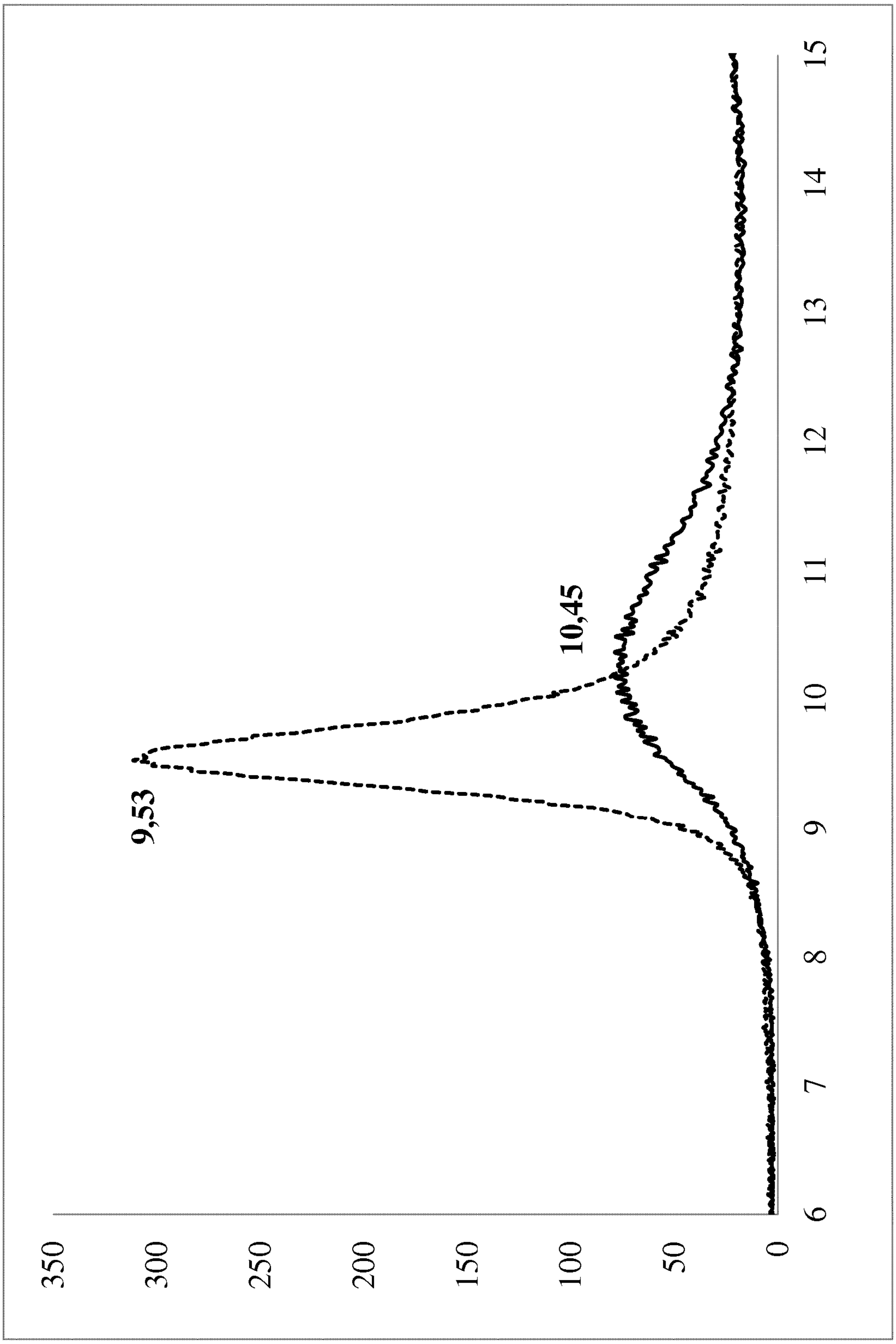


Figure 1

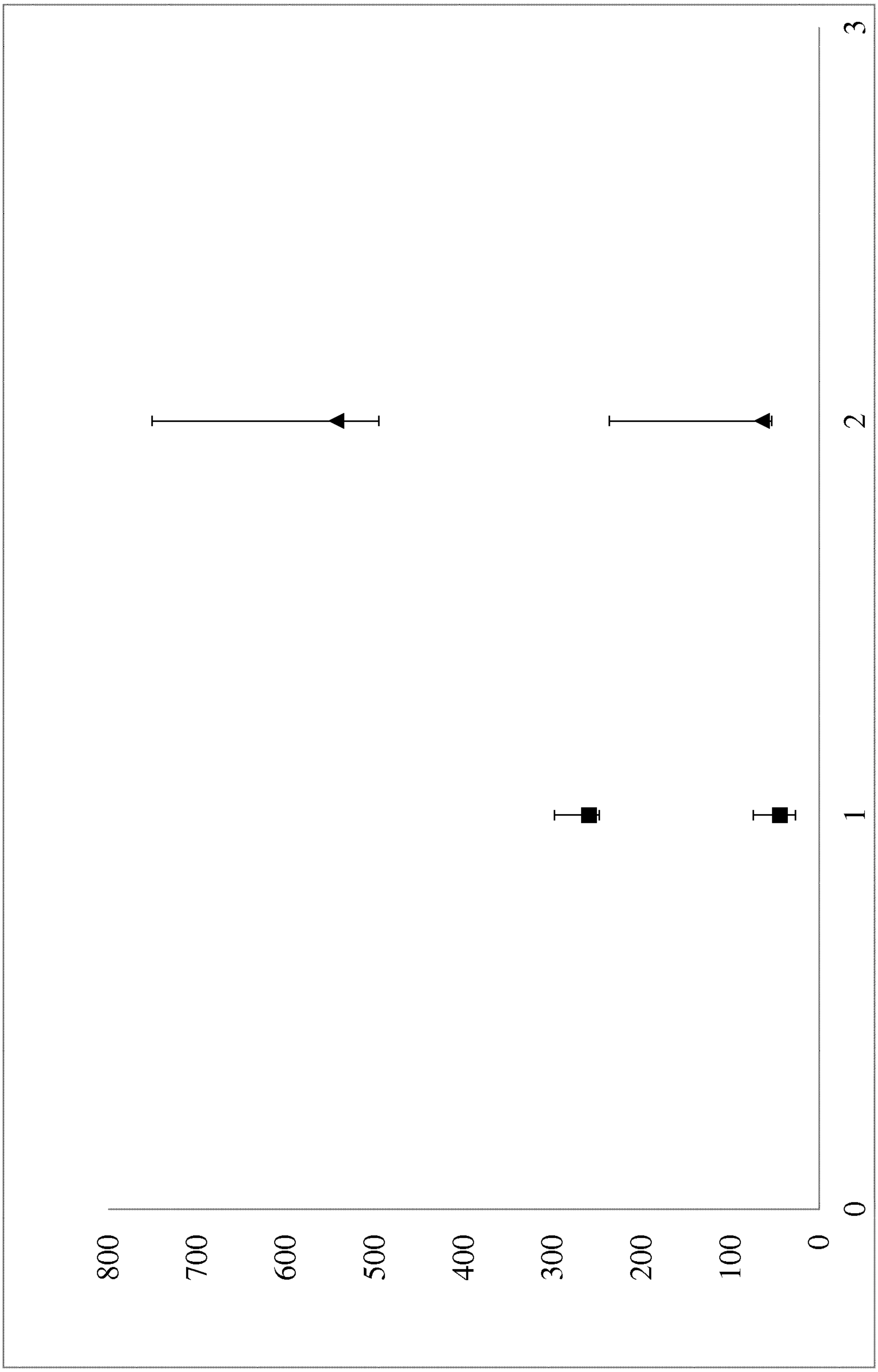


Figure 2

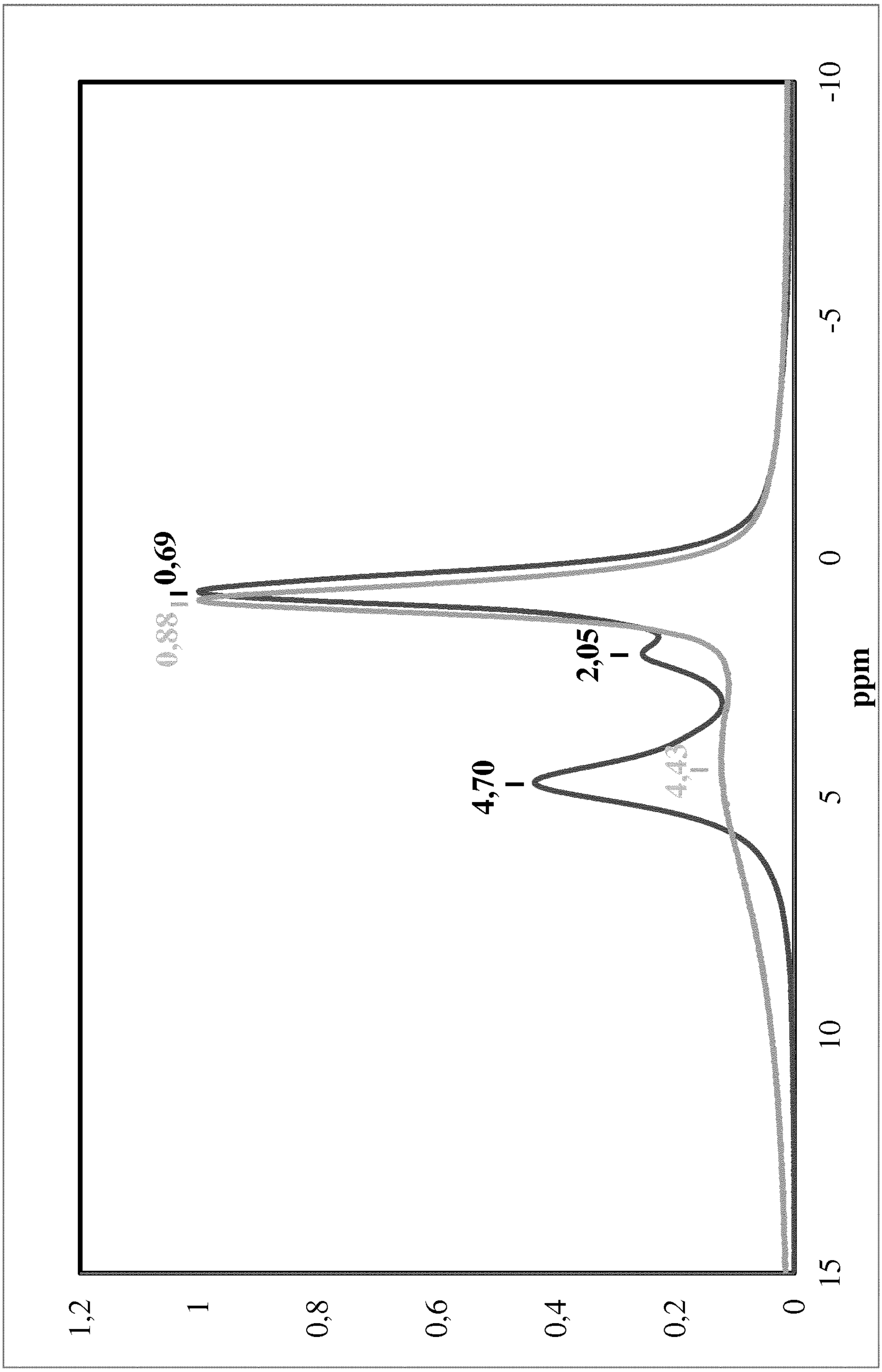


Figure 3

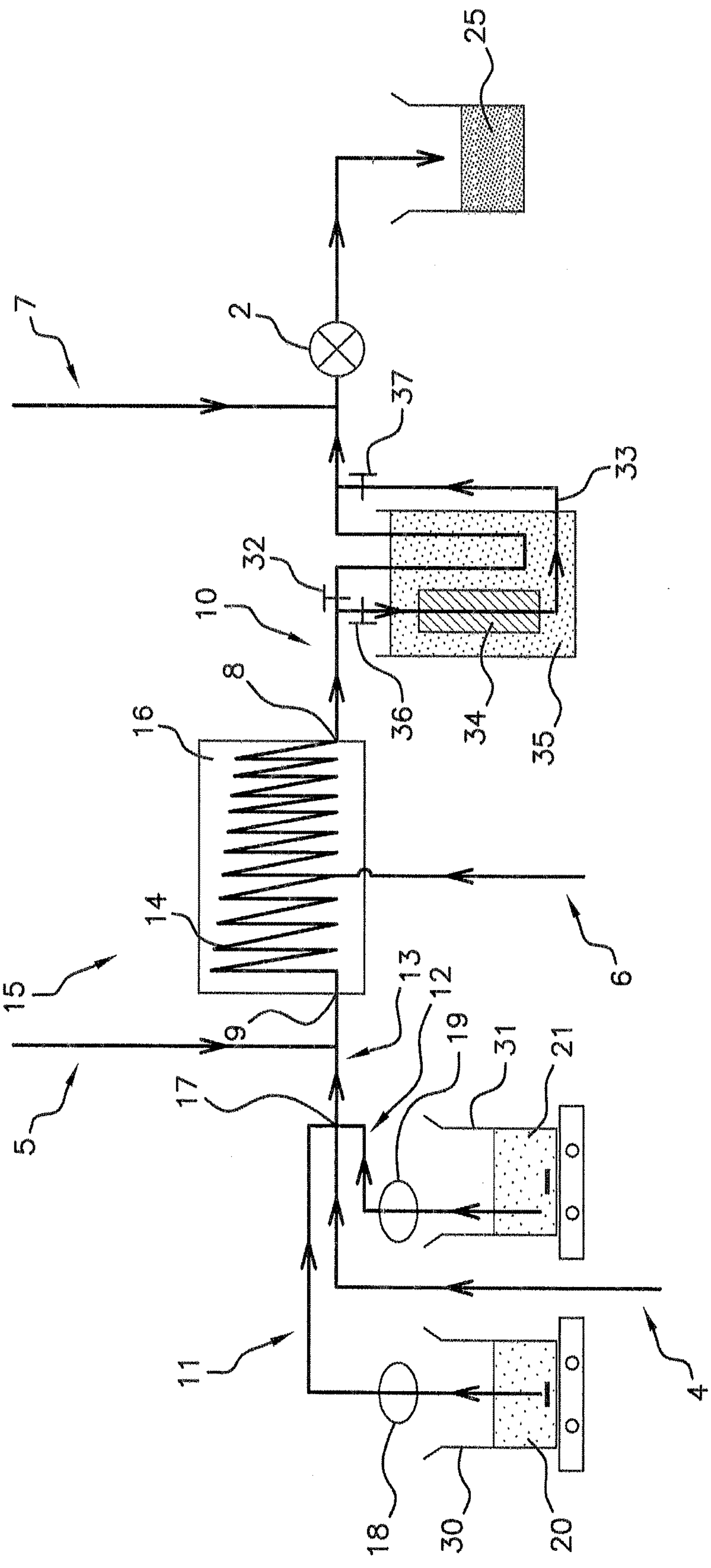


Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014049250 A
- WO 2014207397 A
- WO 2014202920 A
- WO 2016083404 A
- WO 2016083418 A
- FR 2792322
- WO 2013004979 A
- WO 2015159006 A
- FR 1559125
- FR 1559129


### Littérature non-brevet citée dans la description


- **V.M. MARTINEZ.** *Langmuir,* 2004, vol. 20, 5709-5717
- **RAHMAN A. et al.** *Water and Environnement Journal,* 2015, vol. 29, 375-382
- *CHEMICAL ABSTRACTS,* 1064-48-8
- *CHEMICAL ABSTRACTS,* 8005-03-6
- *CHEMICAL ABSTRACTS,* 20262-76-4
- **DE RIEDER et al.** Nomenclature of Micas. *The Canadian Mineralogist,* 1998, vol. 36, 41-48
- *CHEMICAL ABSTRACTS,* 2402-42-8
- *CHEMICAL ABSTRACTS,* 14187-31-6